# FASCICULE DE BREVET EUROPEEN

(11) **EP 3 157 903 B1**
(45) Date de publication et mention de la délivrance du brevet: **11.03.2020**
(21) Numéro de dépôt: 15730141.7
(22) Date de dépôt: 17.06.2015
(51) Int. Cl.: C07D 207/333, C07D 403/10, A61K 31/401, A61P 35/00, C07D 405/10, C07D 207/325

(54) **UTILISATION D'UN NOUVEAU COMPOSE 3-ARYL-4-CATECHOL-PYRROLE-N-PROPANOL ET SES DERIVES POUR LE TRAITEMENT DU CANCER ET DES PATHOLOGIES ASSOCIEES A UNE ANGIOGENESE EXCESSIVE**
VERWENDUNG EINER NEUARTIGEN 3-ARYL-4-CATECHOL-PYRROL-N-PROPANOL-VERBINDUNG UND DERIVATE DAVON ZUR BEHANDLUNG VON KREBS UND ERKRANKUNGEN IM ZUSAMMENHANG MIT ÜBERMÄSSIGER ANGIOGENESE
USE OF A NOVEL 3-ARYL-4-CATECHOL-PYRROLE-N-PROPANOL COMPOUND AND THE DERIVATIVES THEREOF TO TREAT CANCER AND DISEASES RELATED TO EXCESSIVE ANGIOGENESIS

(30) Priorité: 17.06.2014 FR 1455554
(43) Date de publication de la demande: 26.04.2017
(73) Titulaire: Centre National de la Recherche Scientifique (CNRS), 75016 Paris (FR)
(72) Inventeur: COLLIN, Pascal, F-78530 Buc (FR); DELPECH, Bernard, F-92140 Clamart (FR); BAKALA, Joanna, F-75012 Paris (FR); IORGA, Bogdan, F-92160 Antony (FR); FRAPART-JANNAUD, Yves-Michel, F-51240 Ecury sur Coole (FR); PEYROT, Fabienne, F-94200 Ivry (FR); PELISSIER, Franck, F-34400 Saint Christol (FR); ACHAB, Maria Conception, F-91190 Gif S / Yvette (FR)
(74) Mandataire: Lavoix
(86) Numéro de dépôt international: PCT/EP2015/063613
(87) Numéro de publication internationale: WO 2015/193382

(56) Documents cités:
- FR-A1- 2 996 847
- US-A1- 2008 108 648

## Description

### Domaine de l'invention

La présente invention concerne un nouveau composé, le 3-aryl-4-catéchol-N-1*H-*pyrrole et ses dérivés, ainsi que leur procédé de préparation, les compositions pharmaceutiques les contenant et leur utilisation en tant que médicament, notamment dans le traitement du cancer et des pathologies associées à une angiogenèse excessive.

### Art antérieur

Le développement tumoral intègre au moins trois principaux phénomènes biologiques :
1. multiplication incontrôlée des cellules tumorales associée à une inhibition de l'apoptose et une activation des voies de survie et de prolifération,
2. angiogenèse péri-tumorale anormale,
3. présence d'une zone d'hypoxie au centre de la tumeur, à distance des vaisseaux sanguins entrainant des changements phénotypiques des cellules cancéreuses, souvent à l'origine de mécanismes de résistance aux chimiothérapies.

Les traitements anticancéreux actuels qui ciblent le premier phénomène bloquent la multiplication anarchique des cellules cancéreuses, soit par inhibition des facteurs anti-apoptotiques soit par inhibition de la division cellulaire ou du processus de survie. Bien que ces traitements, qui font souvent partie d'une approche multi-thérapeutique, apportent un réel bénéfice dans le traitement des cancers, ils ne sont pas suffisants pour éradiquer toutes les cellules cancéreuses, car ils ne ciblent qu'une partie des mécanismes qui contribuent à la croissance tumorale. A cet inconvénient, s'ajoute le problème de la spécificité de la cible, par rapport aux cellules normales, ainsi que des mécanismes de résistance.

Les recherches récentes ont mis en place une nouvelle approche thérapeutique ayant pour objectif l'inhibition du processus de l'angiogenèse péri-tumorale. En effet, l'angiogenèse est indispensable à la croissance tumorale aussi bien en normoxie qu'en hypoxie. Elle est favorisée par la sécrétion du facteur de croissance vasculaire VEGF par les cellules tumorales, et amplifiée par l'état d'hypoxie en raison de la synthèse du facteur de transcription HIF α. La majorité des drogues anti-angiogéniques utilisées correspond soit à des anticorps dirigés contre le facteur VEGF, soit à des composés dirigés contre le récepteur du VEGF. Malheureusement, les résultats cliniques des thérapies anti-angiogéniques sont très modestes et souvent associés à des mécanismes de résistance et un risque augmenté d'invasion et de métastase. En effet, les vaisseaux péri-tumoraux sont anormaux structurellement et fonctionnellement, ce qui va entrainer une réponse adaptative inverse, aggravant l'anomalie vasculaire et une adaptation des cellules tumorales à ces conditions d'hypoxie augmentée (Nature Review-Cancer ; 10, 417-427, 2011). Des études cliniques sont en cours pour suivre l'effet anti-tumoral de certains agents anti-vasculaires (VDA, vascular-disrupting agents) basé sur ces différences structurales entre vascularisations normale et tumorale. Cependant, une certaine prudence s'impose en raison d'effets cardiovasculaires potentiels. Il apparait donc nécessaire de développer de nouveaux composés anti-angiogéniques ou anti-vasculaires, pour traiter l'angiogenèse péri-tumorale, mais également les pathologies associées à une angiogenèse anormale excessive, comme la DMLA (dégénérescence maculaire liée à l'âge).

Le troisième composant de la croissance tumorale est associé à la diminution de la pression partielle de l'oxygène (pO₂) au centre de la tumeur aboutissant à un état d'hypoxie intratumorale, mentionné ci-dessus. Cet état d'hypoxie se caractérise par des modifications cellulaires métaboliques et génétiques aboutissant à l'activation des voies de signalisation liées à la survie et à l'activation de l'angiogenèse tumorale. Ces modifications sont en partie à l'origine des phénomènes de résistance aux chimiothérapies actuelles. Les drogues actuellement développées pour éliminer les cellules cancéreuses en hypoxie se répartissent en deux groupes : d'une part les prodrogues bio-réductibles par les systèmes enzymatiques à un ou deux électrons, et d'autre part les drogues ciblant les facteurs surexprimés dans la réponse cellulaire à l'état d'hypoxie (HIFα, UPR, thioredoxine), permettant la survie des cellules cancéreuses en hypoxie (Nature Review-Cancer, 11, 393-410, 2011). Les prodrogues bio-réductibles de la première catégorie sont principalement des quinones (α 1-4) (apaziquinone, RH1) ou composés nitrés (TH-302, PR-104), agissant sur l'ADN. L'effet secondaire majeur des composés de la première catégorie mis en évidence au cours des essais cliniques en phase I et II résulte de leur toxicité et leur pénétration extravasculaire limitée, d'autant plus marquée que les cellules hypoxiques sont éloignées des vaisseaux sanguins. Les autres drogues qui agissent sur la voie de la thioredoxine (PX12, motexafin gadolinium) et du facteur de transcription HIF α (PX-478, PX12) sont en cours d'évaluation clinique.

FR 2996847 et US2008/108648 divulguent des composés utiles au traitement des cancers.

A ce jour, chaque drogue utilisée dans la chimiothérapie anticancéreuse ne cible donc qu'un seul phénomène biologique à la fois, et la spécificité des traitements reste faible. Il apparait donc important de trouver de nouveaux composés anti-tumoraux plus spécifiques, agissant simultanément sur les trois composants du développement tumoral.

Les inventeurs de la présente invention ont découvert une nouvelle famille de composés permettant de répondre à ce besoin.

Les inventeurs ont pour cela analysé les données concernant les caractéristiques biologiques, biochimiques et génétiques des cellules cancéreuses en normoxie et hypoxie, pour trouver un dénominateur moléculaire commun, modifié par les cellules cancéreuses ainsi que par les cellules endothéliales associées à l'angiogenèse peri-tumorale anormale.

Les inventeurs ont ainsi découvert que l'analyse de l'ensemble de ces données montre que les cellules cancéreuses, en normoxie et en hypoxie sont caractérisées entre autre par une intense production d'anion superoxyde, causée par une forte activité NADPH oxydases (NOX) (Nature reviews-drug discovery, 8, 579-591, 2009 ; Nature reviews-cancer, 12, 627-637, 2012). Cette surproduction d'anion superoxyde est associée à une dérégulation « redox » générale, associée à une faible activité enzymatique (catalase, peroxyredoxine, glutathion peroxidase) dégradant H₂O₂ ainsi qu'à une forte activation du système thioredoxine (TRX)-thioredoxine reductase (TRX-R), régulant entre autre l'état redox des peroxyredoxines ainsi que l'activité du facteur HIF α. Il existe donc une forte dépendance des cellules cancéreuses vis-à-vis des « espèces réactives dérivées de l'oxygène » (O₂°⁻/ H₂O₂) pour leur survie. Ce stress oxydant est considéré comme un des «talon d'Achille » des cellules cancéreuses. Une surproduction d'anion superoxyde est également retrouvée au niveau des cellules endothéliales actives, en phase de prolifération, causée par une forte activité NADPH oxydases (NOX) au cours de l'angiogenèse. L'isoforme majoritaire des cellules endothéliales est NOX2, régulée par le VEGF, par la voie c-src et Akt, au cours de la phase de prolifération.De plus, les inventeurs ont également découvert que dans le cadre de recherche de pro-drogues anticancéreuses dépourvues d'effets secondaires, cette entité moléculaire commune pourrait également intervenir dans l'activation d'une prodrogue pour former une drogue active pouvant agir sur les trois composants tumoraux, ainsi qu'au cours des pathologies associées à une angiogenèse excessive.

L'anion superoxyde semble donc être un dénominateur commun aux cellules cancéreuses dans les conditions de normoxie et d'hypoxie, ainsi qu'aux cellules endothéliales. De plus, il peut remplir le rôle d'activateur de prodrogue par oxydation.

Les inventeurs ont donc retenu comme critère la surproduction d'anion superoxyde (O₂°⁻) par des cellules tumorales et endothéliales, pour rechercher un nouveau composé (prodrogue) spécifiquement activable par O₂°⁻. Le criblage de la chimiothèque de l'ICSN a permis de sélectionner un composé dont les activités anticancéreuses et anti-angiogeniques se traduisent par :
- inhibition de l'angiogenèse tumorale, sans aucun effet sur l'angiogenèse physiologique ; une inhibition de la croissance « de novo » des tubes vasculaires, une cytotoxicité vis-à-vis des cellules endothéliales uniquement en phase de croissance
- cytotoxicité vis-à-vis des cellules cancéreuses en normoxie, uniquement en présence d' O₂°⁻.
- cytotoxicité vis-à-vis des cellules cancéreuses en hypoxie uniquement en présence d' O₂°⁻.

Afin de déterminer la structure du produit actif obtenu après réaction de la prodrogue avec l'anion superoxyde, le nouveau composé a été exposé *in vitro* à l'anion superoxyde. Il forme par couplage oxydatif un composé dimérique contenant un motif quinone, dont l'activité anti cancéreuse se manifeste par une cytotoxicité vis-à-vis des cellules cancéreuses en normoxie et en hypoxie.

### Description de l'invention

La présente invention concerne les nouveaux composés 3-aryl-4-catechol-pyrrole-N propanol et leurs dérivés de formule générale (I) ci-après, leur préparation, réactivité spécifique vis-à-vis de l'anion superoxyde, leur action sur les cellules cancéreuses en normoxie et hypoxie, sur l'angiogenèse tumorale et physiologique, leur toxicité sur les cellules endothéliales vasculaires, leur action sur la formation de tubes vasculaires ainsi que leur dimérisation par couplage oxydatif et action de ces dimères sur les cellules tumorales, pouvant donc être utilisés pour le traitement du cancer et des pathologies associées à une angiogenèse excessive.

Ainsi, la présente invention a pour objet un nouveau composé, le 3-aryl-4-catéchol-pyrrole-N-propanol et ses dérivés, de formule générale (I) suivante : 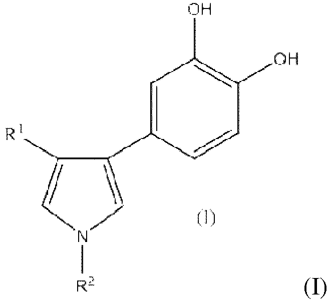 dans laquelle :
- R¹ représente un groupe aryle, en particulier un phényle, éventuellement substitué par un ou plusieurs groupes (C₁-C₂)alkyle, un ou plusieurs halogènes, un ou plusieurs groupes -OH, -CN ou CF₃, et une combinaison de ceux-ci ;et
- R² représente un groupe (C₁-C₆)alkyle ou un groupe hydroxy(C₁-C₆)alkyle ou un groupe (C₁-C₄)alcoxy(C₁-C₆)alkyle ;
ou un hydrate ou solvate pharmaceutiquement acceptable de celui-ci.

Dans la présente invention, on entend par « pharmaceutiquement acceptable » ce qui est utile dans la préparation d'une composition pharmaceutique, qui est généralement sûre, non toxique et ni biologiquement ni autrement non souhaitable, et qui est acceptable pour une utilisation vétérinaire de même que pharmaceutique humaine.

Par « solvates pharmaceutiquement acceptables » d'un composé, on entend désigner dans la présente invention des solvates acceptables pour l'utilisation pharmaceutique des composés selon la présente invention incluant des solvates conventionnels tels que ceux formés, durant la dernière étape du procédé de préparation des composés selon l'invention, avec le(s) solvant(s) de réaction. A titre d'exemple, il peut être fait mention des solvates formés avec l'eau (appelés communément hydrates) ou avec l'éthanol.

Par « aryle », on entend, au sens de la présente invention, un groupe aromatique hydrocarboné, comportant de préférence de 6 à 10 atomes de carbone et comprenant un ou plusieurs, notamment 1 ou 2, cycles accolés, comme par exemple un groupement phényle ou naphtyle. Avantageusement, il s'agit du phényle.

Par groupement « (C₁-C₆)alkyle », on entend, au sens de la présente invention, une chaîne hydrocarbonée saturée, linéaire ou ramifiée, comportant 1 à 6, en particulier 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par groupement « (C₁-C₂)alkyle », on entend, au sens de la présente invention, une chaîne hydrocarbonée saturée, linéaire ou ramifiée, comportant 1 à 2 atomes de carbone. A titre d'exemple, on peut citer les groupes méthyle et éthyle.

Par groupement « (C₁-C₄)alcoxy », on entend, au sens de la présente invention, un groupe (C₁-C₄)alkyle, tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore tert-butoxy.

Par groupe « hydroxy-(C₁-C₆)alkyle », on entend, au sens de la présente invention, un groupement hydroxyle (-OH), lié à la molécule par l'intermédiaire d'un groupe (C₁-C6)alkyle, tel que défini ci-dessus. A titre d'exemple, on peut citer le groupement 3-hydroxypropyle.

Par groupe « (C₁-C₄)alcoxy-(C₁-C₆)alkyle, on entend, au sens de la présente invention, un groupement (C₁-C₄)alcoxy, tel que défini ci-dessus, lié à la molécule par l'intermédiaire d'un groupe (C₁-C₆)alkyle, tel que défini ci-dessus. A titre d'exemple on peut citer le groupe 3-éthoxypropyle.

Par « atome d'halogène », on entend, au sens de la présente invention, les atomes de fluor, de chlore, de brome et d'iode.

Dans un mode de réalisation avantageux,
- R¹ représente un phényle, éventuellement substitué par un ou plusieurs groupes (C₁-C₂)alkyle, un ou plusieurs halogènes, un ou plusieurs groupes -OH, -CN ou CF₃, et une combinaison de ceux-ci ; et
- R² représente un groupe (C₁-C₆)alkyle ou un groupe hydroxy(C₁-C₆)alkyle ou un groupe (C₁-C₆)alcoxy(C₁-C₆)alkyle ;

De manière avantageuse, R¹ représente un phényle et R² représente un groupe (C₁-C₆)alkyle ou un groupe hydroxy(C₁-C₆)alkyle ou un groupe (C₁-C₄)alcoxy(C₁-C₆)alkyle.

De manière plus avantageuse, R¹ représente un phényle et R² représente un groupe (C₁-C₄)alkyle ou un groupe hydroxy(C₁-C₄)alkyle ou un groupe (C₁-C₂)alcoxy(C₁-C₄)alkyle.

Dans un mode de réalisation particulier, R¹ représente un phényle et R² représente un groupe hydroxy(C₁-C₆)alkyle.

En particulier, les composés de l'invention pourront être choisis parmi les composés de formules (Ia), (Ib) et (Ic) suivantes et leurs solvates et hydrates pharmaceutiquement acceptables, tels que le solvate obtenu avec le DMSO.

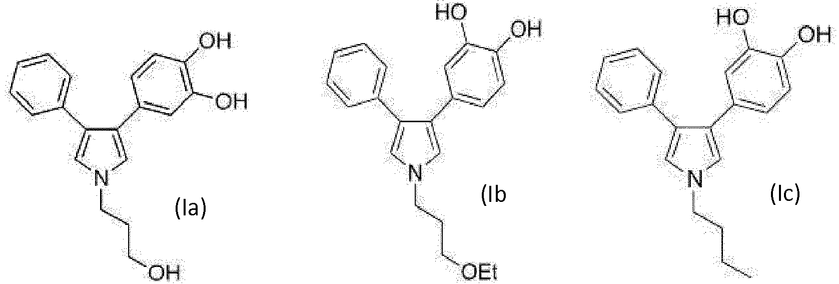

L'invention a également pour objet un composé de formule générale (I) suivante : 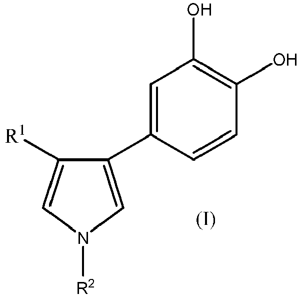 dans laquelle :
- R¹ représente un groupe aryle, en particulier un phényle, éventuellement substitué par un ou plusieurs groupes (C₁-C₂)alkyle, un ou plusieurs halogènes, un ou plusieurs groupes -OH, -CN ou CF₃, et une combinaison de ceux-ci ; et
- R² représente un groupe (C₁-C₆)alkyle ou un groupe hydroxy(C₁-C₆)alkyle ou un groupe (C₁-C₄)alcoxy(C₁-C₆)alkyle ;
ou un hydrate ou solvate pharmaceutiquement acceptable de celui-ci,
pour son utilisation en tant que médicament, notamment destiné au traitement du cancer.

Le composé de formule générale (I) selon l'invention utilisé comme médicament pourra être choisi parmi les composés de formule (Ia), (Ib) et (Ic) suivantes :

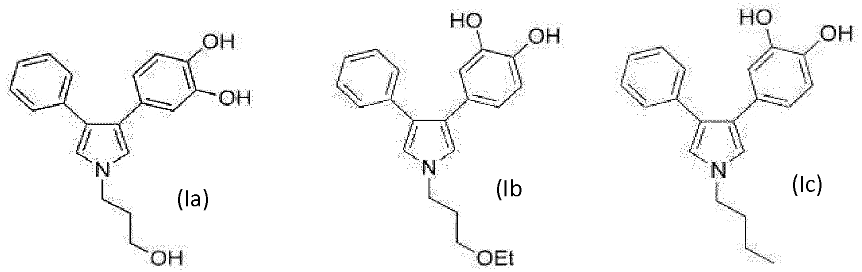

Selon un aspect de l'invention, le composé de formule générale (I) selon l'invention peut se dimériser, notamment par couplage oxydatif. En particulier, la dimérisation du composé selon l'invention est provoquée en présence d'anion superoxyde.

Ainsi, la présente invention a également pour objet le composé de formule générale (I) selon l'invention en tant que prodrogue. Il est décrit que ladite prodrogue, non toxique, sera activée par sa dimérisation par intéraction avec l'anion superoxyde.

Dans un mode de réalisation particulier, on décrit le composé selon l'invention pour son utilisation en tant que prodrogue activée par dimérisation, notamment par couplage oxydatif, en particulier dans le traitement du cancer et des pathologies associées à une angiogenèse excessive.

La dimérisation s'effectue de préférence en présence d'anion superoxyde et conduit de préférence à au moins un des composés de formule générale (II), (III) ou (IV) : 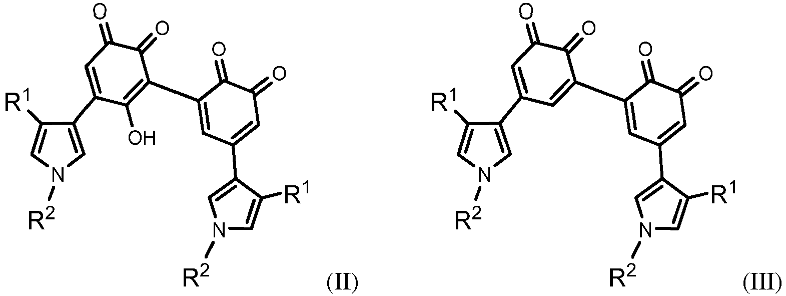 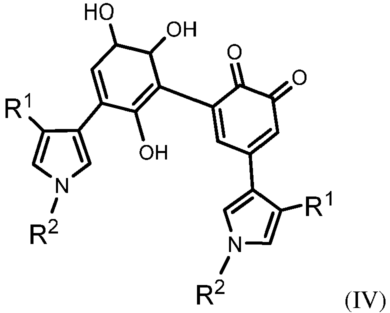 dans laquelle R¹ et R² sont tels que définis ci-dessus,
ou un hydrate ou solvate pharmaceutiquement acceptable de celui-ci.

La dimérisation peut conduire à un composé de formule générale (II) ou à un composé de formule (IIa) suivante :

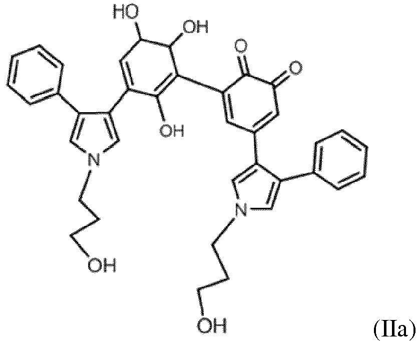

La présente invention a également pour objet le composé de formule générale (IV) ci-dessous 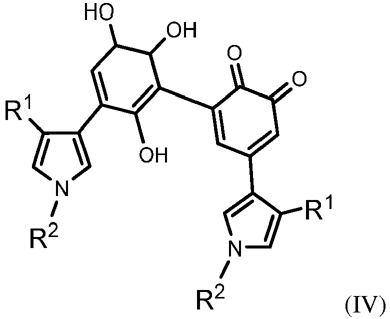 dans laquelle R¹ et R² sont tels que définis ci-dessus, ou un hydrate ou solvate pharmaceutiquement acceptable de celui-ci.

Avantageusement, le dimère de formule générale (II) comme décrit ci-dessus est le composé de formule (IIa).

La présente invention porte également sur le composé de formule générale (IV), dans laquelle R¹ et R² sont tels que définis ci-dessus, ou un hydrate ou solvate pharmaceutiquement acceptable de celui-ci, pour son utilisation en tant que médicament.

La présente invention concerne également l'utilisation d'un composé de formule (I) ou (IV) selon l'invention pour la fabrication d'un médicament, notamment destiné au traitement du cancer.

Aussi décrite est une méthode de traitement du cancer, comprenant l'administration d'une quantité efficace d'au moins un composé de formule (I) ou (IV) selon l'invention à un patient en ayant besoin.

Le cancer peut être notamment les tumeurs solides ou non solides, tels que le mélanome, le cancer colorectal, le cancer du poumon, le cancer de la prostate, le cancer du foie, le cancer du sein, le cancer de l'utérus, le cancer de l'estomac, le cancer du pancréas, le cancer de la vessie, le cancer de l'ovaire, les cancers de la tête et du cou, le cancer du cerveau, la leucémie, les lymphomes (dont le lymphome de Burkitt) et les myélomes.

La présente invention a également pour objet une composition pharmaceutique comprenant au moins un composé de formule générale (I) ou (IV) suivantes : 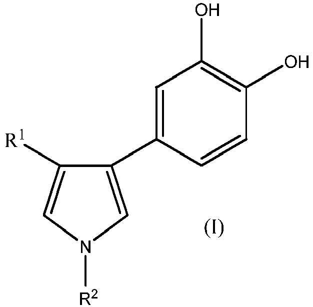 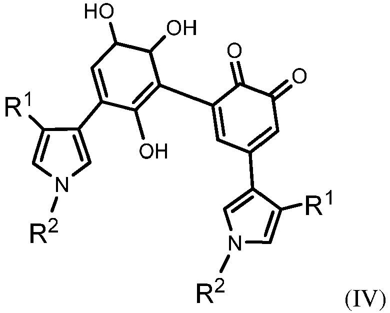 dans laquelle :
R¹ et R² sont définis comme ci-avant
ou un hydrate ou solvate pharmaceutiquement acceptable de celui-ci,
et un excipient pharmaceutiquement acceptable.

Les composés selon l'invention peuvent être administrés par voie orale, sublinguale, parentérale, sous-cutanée, intramusculaire, intraveineuse, transdermique, locale ou rectale.

Dans les compositions pharmaceutiques de la présente invention pour l'administration orale, sublinguale, parentérale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration parentérale, sous-cutanée, intramusculaire, intraveineuse, intranasale ou intraoculaire et les formes d'administration rectale.

Les composés selon l'invention peuvent être couplés avec un anticorps qui cible spécifiquement un marqueur tumoral (par exemple une protéine qui, dans l'idéal, ne doit être trouvé que dans ou sur les cellules tumorales) afin de former un conjugué anticorps-médicament (ADC = Antibody-Drug Conjugate). De même, les composés selon l'invention peuvent être couplés avec tout autre vecteur permettant de cibler les cellules cancéreuses. Parmi ces vecteurs, on peut citer les vecteurs viraux ou issus d'êtres vivants, tels que les rétrovirus, les vecteurs synthétiques, tels que les liposomes ou des nanoparticules et en particulier des nanoparticules de dioxide de titane, ou des méthodes physiques telles que l'électroporation.

Les composés de l'invention peuvent être utilisés à des doses dépendant de l'effet recherche, de la durée du traitement et de la voie d'administration utilisée. L'homme du métier choisira également la voie d'administration et le dosage les mieux adaptés en fonction du sujet et de la maladie à traiter.D'une façon générale, le médecin déterminera la posologie appropriée en fonction de l'âge, du poids et de tous les autres facteurs propres au sujet à traiter.

L'excipient pharmaceutiquement acceptable est connu de l'Homme du Métier et est choisi selon le mode d'administration de la composition pharmaceutique. A titre d'exemple, l'excipient pharmaceutiquement acceptable peut être choisi dans le groupe constitué par les agents diluants, liants, désintégrants, les colorants, les lubrifiants, les agents solubilisants, les agents promoteurs d'absorption, les filmogènes, les agents gélifiants, et leurs mélanges.

La composition pharmaceutique telle que décrite ci-dessus peut être utile dans le traitement du cancer.

La présente invention concerne également l'utilisation d'une composition pharmaceutique telle que décrite ci-dessus pour la fabrication d'un médicament destiné au traitement du cancer.

Aussi décrite est une méthode de traitement du cancer, comprenant l'administration d'une quantité efficace d'une composition pharmaceutique de l'invention à un patient en ayant besoin.

Le composé de formule générale (I) selon l'invention utilisé dans les compositions pharmaceutiques selon l'invention pourra être choisi parmi les composés de formule (Ia), (Ib) et (Ic) suivantes :

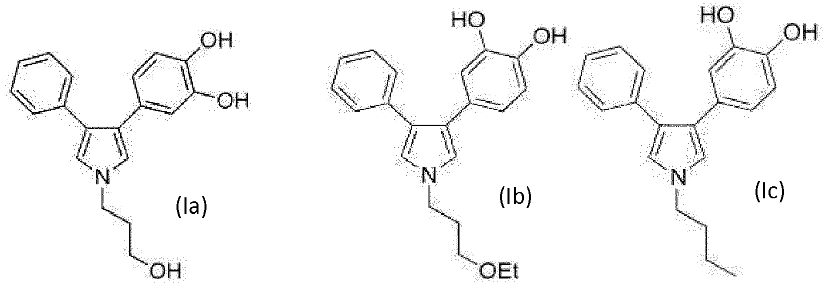

La présente invention a également pour objet un procédé de préparation d'un composé de formule (I) suivante : 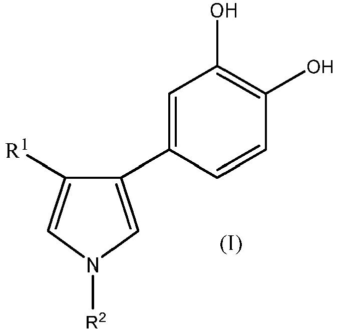 dans laquelle :
- R¹ représente un groupe aryle, en particulier un phényle, éventuellement substitué par un ou plusieurs groupes (C₁-C₂)alkyle, un ou plusieurs halogènes, un ou plusieurs groupes -OH, -CN ou CF₃, et une combinaison de ceux-ci.
- R² représente un groupe (C₁-C₆)alkyle ou un groupe hydroxy(C₁-C₆)alkyle ou un groupe (C₁-C₄)alcoxy(C₁-C₆)alkyle ;
ou un hydrate ou solvate pharmaceutiquement acceptable de celui-ci,
comprenant, en tant qu'étape clé, la réaction de condensation d'un composé de formule (V) suivante : 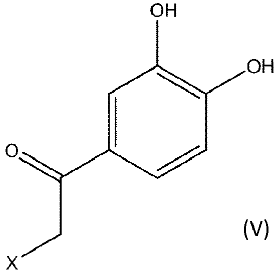 dans laquelle X est un halogène, en particulier un atome de chlore,
avec un composé de formule (VI) et un composé de formule (VII) suivantes :

R²-NH₂ (VI)

R¹-CH₂-CHO (VII)

dans laquelle R¹ et R² sont tels que défini ci-dessus.

Dans un mode de réalisation préféré, les composés selon l'invention sont avantageusement préparés dans les conditions réactionnelles décrites dans les schémas 1 à 5 ci-dessous.

Les procédés de préparation des composés selon l'invention peuvent éventuellement comprendre des réactions supplémentaires de protection et/ou de déprotection afin d'éviter des réactions secondaires bien connues de l'homme du métier. Celui-ci dispose d'un vaste choix de groupes protecteurs qui sont notamment décrits par Greene T.W. et coll., Protecting Groups in Organic Synthesis, third édition, 1999, John Wiley & sons.

Les composés selon l'invention peuvent en outre être purifiés par des méthodes connues de l'homme du métier. On peut citer, par exemple, les méthodes de purification par cristallisation, par chromatographie ou par extraction.

### Description des Figures

Figure 1. Étude de la réactivité du composé (1) avec l'anion superoxyde par une méthode de compétition avec le piégeur BMPO et un suivi par résonance paramagnétique électronique (RPE). L'anion superoxyde est produit en phase organique par la dissolution du superoxyde de potassium (KO2) dans le DMSO (A) et en phase aqueuse par la réaction enzymatique de la xanthine oxydase (XO) avec la xanthine (X) (B).
Figure 2. Etude par résonance paramagnétique électronique (RPE) de la réaction du composé (1) avec l'anion superoxyde, provenant de la dissolution par sonication de KO2 dans le DMSO : A - Suivi de la diminution du signal RPE de O2°- à température de l'azote liquide 77 K) en fonction de la concentration du composé (1) ajouté ; B- spectre RPE de l'intermédiaire radicalaire observé à température ambiante et sa simulation.
Figure 3. Formation d'une quinone intermédiaire après réaction du composé (1) avec l'anion superoxyde (KO2) en phase organique (DMSO). Analyse par HPLC-MS sur LC-TRAP (A) et par RMN 500MHz (C13) (B).
Figure 4. Formation d'un composé dimérique par couplage oxydatif en présence (A) d'anion superoxyde produit par la xanthine oxydase (analyse par HPLC-TRAP) ou (B) à pH égal au pKa (8.89) (analyse par UHPLC). Spectre UV du composé dimerique MH+ 631 (C) obtenu par couplage oxydatif à pH 9.4.
Figure 5. (A) Effet inhibiteur du composé *(1)* sur l'angiogenèse tumorale (glioblastome humain greffé sur la membrane chorioallantoïdienne de l'embryon de poulet). (B) Etude de l'effet dose-dépendant du composé (*1*) sur la sécrétion de VEGF par les cellules U87 et HCT-116 en condition de normoxie et d'hypoxie (2% O₂).
Figure 6. (A) Absence de l'effet du composé *(1)* sur l'angiogenèse physiologique étudiée *in ovo* sur le modèle de la membrane chorioallantoïdienne de l'embryon de poulet. (B) Effet du composé (*1*) sur la formation de tubes vasculaires, à partir de cellules endothéliales cultivées sur matrigel. (C) Effet du composé *(1)* sur la survie de cellules endothéliales (HUVEC) cultivées en phase de prolifération ou de quiescence pendant 48h.
Figure 7. Evaluation de l'activité biologique du composé *(1)* en normoxie (A) Effet du composé *(1)* sur la croissance et la cytotoxicité (IC50) des cellules cancéreuses HCT 116, U87, MDA-MB-231, A549, MCF-7, K562 et K562 R après 72h de traitement, (B) Effet des composés (2), (3), (4), et (5) sur la croissance des cellules HCT-116 après 72h de traitement, en normoxie.
Figure 8. Effets du composé *(1)* et du composé dimérique MH+= 631 sur la croissance (IC50) des cellules cancéreuses HCT-116 après 48h de traitement en normoxie (A) et en hypoxie (B).
Figure 9. Effet du composé *(1)* sur l'activité des caspases 3 et 7 évalué sur les cellules cancéreuses HCT-116, U87 et HL60 en normoxie
Figure 10. Suppression du signal de l'anion superoxyde cellulaire des cellules HCT-116 par le composé *(1)* en normoxie. Analyse par HPLC-Fluorimétrie en présence de la sonde dihydroethidium (DHE). Trait continu : contrôle ; trait pointillé : traité par le composé (*1*)
Figure 11. Dépendance du composé *(1)* vis-à-vis de l'anion superoxyde extracellulaire/membranaire pour les effets cytotoxiques en normoxie et hypoxie (A) ; pour les effets pro-apoptotiques en normoxie : condensation de la chromatine (B) et activité caspase 3-7 (C) : effet de la SOD à 50U/ml

L'invention sera mieux comprise à la lumière des exemples qui suivent.

### EXEMPLES

Abréviations utilisées :
- AcOEt: Acétate d'éthyle
- BMPO: 5-tert-butoxycarbonyl-5-methyl-1-pyrroline-*N*-oxide
- CI50: Concentration inhibitrice médiane
- DHE: Dihydroéthidium
- DMSO: Diméthylsulfoxyde
- DPBS: Dulbecco's Phosphate-Buffered Saline
- HClO₄: Acide perchlorique
- HPLC: chromatographie liquide haute performance
- Na₂SO₄: Sulfate de sodium
- NaCl: Chlorure de sodium
- NaHCO₃: Bicarbonate de sodium
- NaI: Iodure de sodium
- PBS: Phosphate buffered saline
- RMN: Résonnance magnétique nucléaire
- RPE: Résonnance paramagnétique électronique
- SOD: Superoxyde dismutase
- TA: Température ambiante
- THF: Tétrahydrofurane

### EXEMPLE 1 : Synthèse des composés de l'invention

### 4-(1-(3-Hydroxypropyl)-4-phényl-1H-pyrrole-3-yl)benzène-1,2-diol) (1).

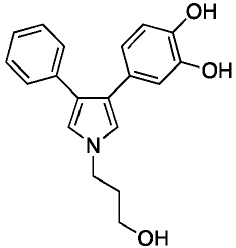

Le composé 4-(1-(3-hydroxypropyl)-4-phényl-1*H*-pyrrole-3-yl)benzène-1,2-diol) (*1*) a été préparé via la condensation de la 2-chloro-3',4'-dihydroxyacétophénone avec le 3-aminopropan-1-ol et le phénylacétaldéhyde suivant le Schéma 1.

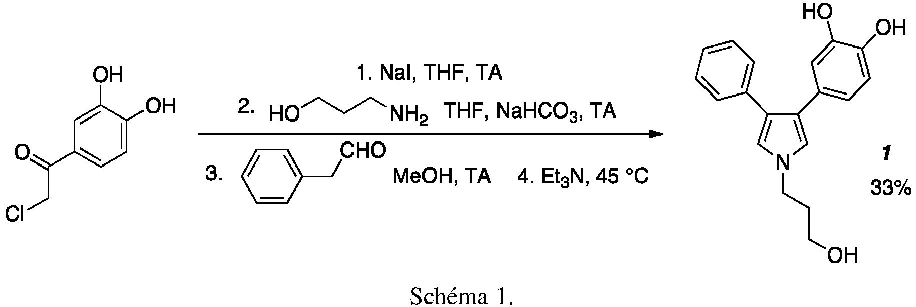

Un mélange de 2-chloro-3',4'-dihydroxyacétophénone (785 mg, 4,2 mmol) et de NaI (3,15 g, 21 mmol) dans du THF (20 mL) est agité pendant 25 min à TA. Du 3-aminopropan-1-ol (320 µL, 4,2 mmol) puis NaHCO₃ (1,5 g, 18 mmol) sont ajoutés et le mélange est agité pendant 1 h à TA. Une solution de phénylacétaldéhyde (500 µL, 4,2 mmol) dans du méthanol (30 mL) est ajoutée et le mélange est agité pendant 30 min. De la triéthylamine (600 µL, 4,2 mmol) est ajoutée et le mélange est agité pendant 16 h à 45 °C. Le produit est extrait par un mélange éther/chloroforme 5:1 et la phase organique est lavée avec une solution de NaHCO₃/NaCl et de la saumure, séchée sur Na₂SO₄, évaporée sous pression réduite et chromatographiée sur gel de silice (élution avec un gradient de pentane à THF) pour donner 428 mg (33%) de pyrrole *1* sous la forme de semi-solide amorphe. RMN ¹H (CD₃OD; 300 MHz) *δ*(ppm) 7,15-6,92 (5 H; m); 6,68 (1 H; d; *J* = 2,4 Hz); 6,59 (1 H; d; *J* = 2,4 Hz); 6,56-6,50 (2 H; m); 6,44 (1 H; dd; *J* = 8,1; 2,1 Hz); 3,89 (2 H; t; *J* = 6,9 Hz); 3,48 (2 H; t; *J* = 6,9 Hz); 1,89 (2 H; quint; *J* = 6,9 Hz). RMN ¹³C (CD₃OD; 75 MHz) *δ* (ppm) 145,8; 144,4; 138,0; 129,9; 129,2; 129,0; 126,1; 124,5; 124,1; 121,2; 121,1; 121,0; 117,0; 116,1; 59,7; 47,0; 35,3. IR (neat) *v*ₘₐₓ cm⁻¹ 3337; 2946; 1599; 1542; 1435; 1395; 1354; 1286; 1257; 1190; 1163; 1111; 1057; 770; 700. HRMS (ES) (*m*/*z*)[M+H]⁺ calculé pour C₁₉H₂₀NO₃ 310,1443; trouvé 310,1457.

### 3-(3-(2,3-Dihydrobenzo[b][1,4]dioxin-6-yl)-4-phényl-1H-pyrrole-1-yl)propan-1-ol (2).

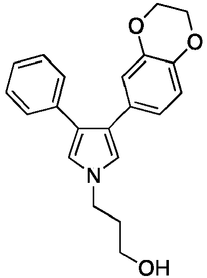

Le composé 3-(3-(2,3-dihydrobenzo[*b*][1,4]dioxin-6-yl)-4-phényl-1*H*-pyrrole-1-yl)propan-1-ol (2) a été préparé via la condensation du 6-chloroacétyl-1,4-benzodioxane avec le 3-aminopropan-1-ol et le phénylacétaldéhyde suivant le Schéma 2.

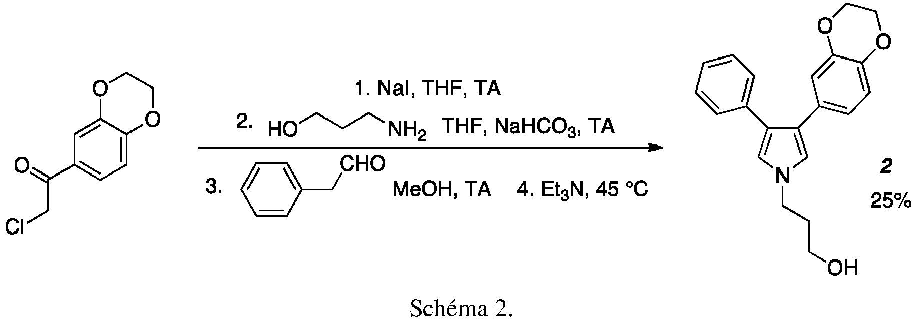

Un mélange de 6-chloroacétyl-1,4-benzodioxane (1,0 g, 4,56 mmol) et de NaI (3,453 g, 22,8 mmol) dans du THF (23 mL) est agité pendant 25 min à TA. Du 3-aminopropan-1-ol (350 µL, 4,56 mmol) puis NaHCO₃ (1,64 g, 19,6 mmol) sont ajoutés et le mélange est agité pendant 1 h à TA. Une solution de phénylacétaldéhyde (566 µL, 4,56 mmol) dans du méthanol (32 mL) est ajoutée et le mélange est agité pendant 30 mn. De la triéthylamine (636 µL, 4,56 mmol) est ajoutée et le mélange est agité pendant 16 h à 45 °C. Le produit est extrait par un mélange éther/chloroforme 5:1 et la phase organique est lavée avec une solution de NaHCO₃/NaCl et de la saumure, séchée sur Na₂SO₄, évaporée sous pression réduite et chromatographiée sur gel de silice (heptane/AcOEt 6:4) pour donner 394 mg (25%) de pyrrole 2.

### 3-(3,4-diphenyl-1H-pyrrol)propan-1-ol (3).

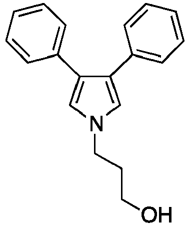

Le composé 3-(3,4-diphenyl-1*H*-pyrrol)propan-1-ol (3) a été préparé via la condensation de la 2-bromoacétophénone avec le 3-aminopropan-1-ol et le phénylacétaldéhyde suivant le schéma 3.

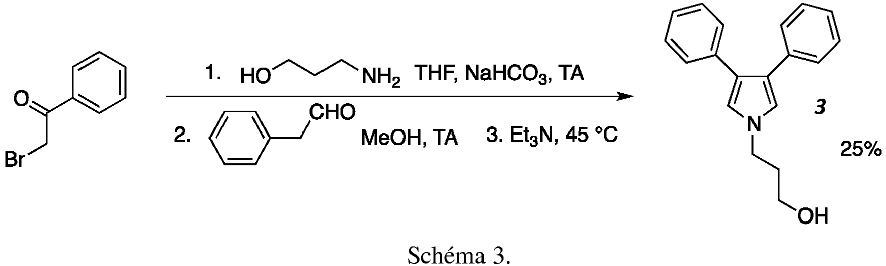

A une solution de 2 bromoacétophenone (597,2 mg; 3 mmol) dans 15 mL de THF agitée à température ambiante, du 3-aminopropan-1-ol (233,9 µL; 3 mmol) puis de l'hydrogénocarbonate de sodium (1,086 g; 12,9 mmol) sont ajoutés. Après 1 heure d'agitation à température ambiante, une solution de phénylacétaldéhyde 90 % (390 µL; 3 mmol) dans 21,4 mL de méthanol est additionnée goutte-à-goutte. Après 30 minutes d'agitation de la triéthylamine (418,2 µL; 3 mmol) est additionnée. La solution résultante est agitée à 45 °C pendant 20 h. Après addition d'eau, le produit est extrait avec du dichlorométhane. Un lavage de la phase organique avec un mélange 1/1 de solutions saturées en NaHCO₃ et NaCl, suivi d'un séchage au MgSO₄ puis d'une évaporation et d'un placement sous le vide d'une pompe à palettes permet d'obtenir le produit brut sous forme d'une huile orangée (881mg). Après chromatographie sur silice (60A empâtée heptane/AcOEt 1/1, colonne de 25 cm de hauteur, diamètre 2 cm, introduction acétate d'éthyle) les fractions collectées permettent d'estimer par RMN un rendement de l'ordre de 25%. La fraction pure reprise à l'acétonitrile et lavée au pentane permet d'obtenir 44 mg de produit pur (huile orangée) pour les analyses. RMN ¹H (CDCl₃; 500 MHz) *δ* (ppm) 2,05 (m; 2 H); 3,70 (t; *J* = 6,0 Hz; 2 H); 4,05 (t; *J* = 6,9 Hz; 2 H); 6,79 (sl; 2 H); 7,18 (m; 2 H); 7,27 (m; 4 H), 7,31 (m; 4 H). RMN ¹³C(CDCl₃; 125 MHz) *δ* (ppm) 33,9; 46,3; 59,6; 120,4; 123,3; 125,6; 128,2; 128,5; 136,1. IR (film) *v*ₘₐₓ cm⁻¹ 3315; 3057; 2932; 1697; 1601. MS (ES⁺) *(m*/*z)* 278 (100%, [M+H]⁺). HRMS (ES) (*m*/*z*) (278,1542; [M+H]⁺); calculée pour C₁₉H₂₀NO: 278,1545.

### 4-(1-(3-Éthoxypropyl)-4-phényl-1H-pyrrole-3-yl)benzène-1,2-diol (4).

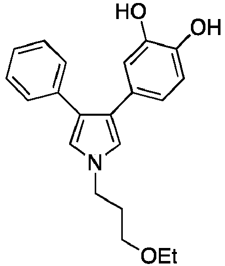

Le composé 4-(1-(3-éthoxypropyl)-4-phényl-1*H*-pyrrole-3-yl)benzène-1,2-diol (4) a été préparé via la condensation de la 2-chloro-3',4'-dihydroxyacétophénone avec la 3-éthoxypropylamine et le phénylacétaldéhyde suivant le Schéma 4.

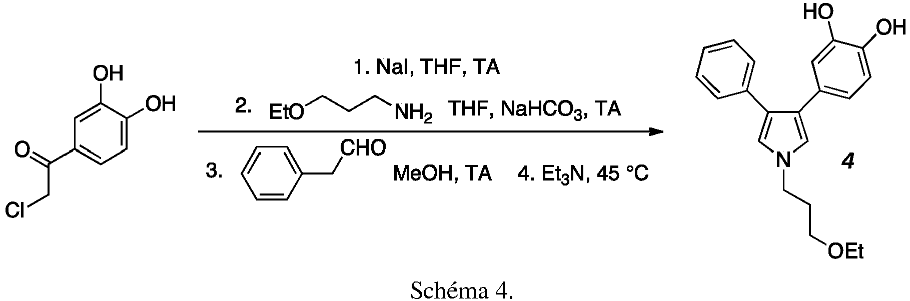

Un mélange de 2-chloro-3',4'-dihydroxyacétophénone (1,0 g, 5,2 mmol) et de NaI (3,94 g, 26 mmol) dans du THF (25,9 mL) est agité pendant 25 min à TA. De la 3-éthoxypropylamine (630 µL, 5,2 mmol) puis NaHCO₃ (1,88 g, 22,4 mmol) sont ajoutés et le mélange est agité pendant 1 h à TA. Une solution de phénylacétaldéhyde (650 µL, 5,2 mmol) dans du méthanol (37 mL) est ajoutée et le mélange est agité pendant 30 min. De la triéthylamine (720 µL, 5,2 mmol) est ajoutée et le mélange est agité pendant 16 h à 45 °C. Le produit est extrait par un mélange éther/chloroforme 5:1 et la phase organique est lavée avec une solution de NaHCO₃/NaCl et de la saumure, séchée sur Na₂SO₄, évaporée sous pression réduite et chromatographiée sur gel de silice.

### 4-(1-(3-Hydroxypropyl)-4-butyl-1H-pyrrole-3-yl)benzène-1,2-diol) (5).

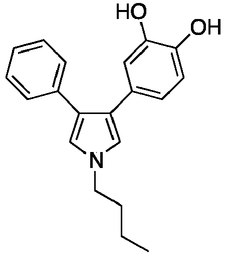

Le composé 4-(1-butyl-4-phényl-1*H*-pyrrole-3-yl)benzène-1,2-diol) (*1*) a été préparé via la condensation de la 2-chloro-3',4'-dihydroxyacétophénone avec le 3-aminopropan-1-ol et le phénylacétaldéhyde suivant le schéma suivant.

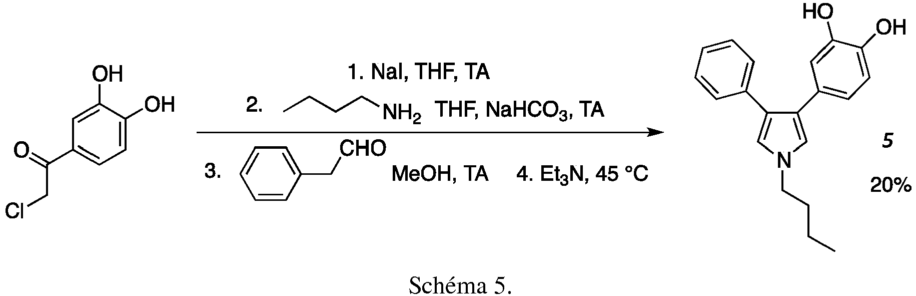

Un mélange de 2-chloro-3',4'-dihydroxyacétophénone (279,9 mg, 1,5 mmol) et de NaI (224,9 mg, 1,5 mmol) dans du THF (7,5 mL) est agité pendant 30 min à TA. De la butylamine (148 µL, 1,5 mmol) puis NaHCO₃ (542,8 mg, 6,46 mmol) sont ajoutés et le mélange est agité pendant 1 h à TA. Une solution de phénylacétaldéhyde à 90% (195 µL, 1,5 mmol) dans du méthanol (10,7 mL) est ajoutée goutte-à-goutte et le mélange est agité pendant 30 min. De la triéthylamine (209 µL, 1,5 mmol) est ajoutée et le mélange est agité pendant 20 h à 45 °C. Après addition d'eau, le produit est extrait par un mélange éther/chloroforme 5:1 et la phase organique est lavée avec une solution de NaHCO₃ sat./NaCl sat. 1:1 et séchée sur Na₂SO₄, évaporée sous pression réduite pour donner 511 mg d'huile noire contenant 20% de pyrrole *1* (estimation par RMN). Après chromatographie sur gel de silice (élution avec un gradient de heptane/acétate d'éthyle 7:3 → 1:1) le produit a été purifié par HPLC. Un échantillon pur de *1* a été obtenu sous la forme d'huile orangée. RMN ¹H (CDCl₃; 500 MHz) *δ*(ppm) 7,19-7,33 (4 H; m); 7,15 (1 H; m); 6,66-6,78 (5 H; m); 3,88 (2 H; t; *J* = 7,6 Hz); 1,81 (2 H; quint; *J* = 7,6 Hz); 1,40 (2 H; sext; *J* = 7,6 Hz); 0,96 (3 H; t; *J* = 7,6 Hz). RMN ¹³C (CDCl₃; 125 MHz) *δ* (ppm) 143,1; 141,8; 136,2; 129,6; 128,5 (2 C); 128,3 (2 C); 125,6; 122,9; 122,6; 121,5; 120,2; 120,0; 115,7; 115,4; 49,7; 33,6; 20,2; 13,8. IR (neat) *v*ₘₐₓ cm⁻¹ 3372; 2958; 1673; 1601. MS (ES⁻) (*m*/*z*) 306 [M-H]⁻. HRMS (ES) (*m*/*z*) [M-H]⁻ calculé pour C₂₀H₂₀NO₂ 306,1494; trouvé 306,1483.

### EXEMPLE 2 : Réactivité du composé (1) avec l'anion superoxyde in vitro ; étude par résonance paramagnétique électronique (RPE) en présence de BMPO. Détermination de la CI50

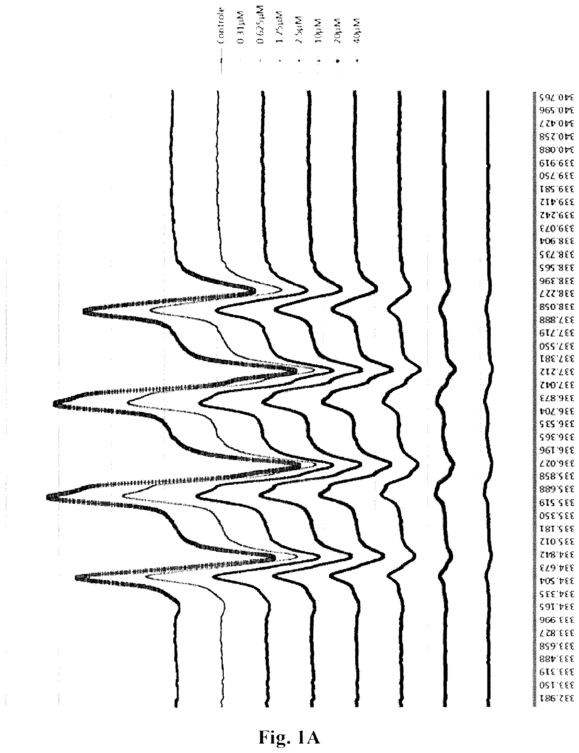

La réactivité du composé (1) avec l'anion superoxyde en phase organique a été étudiée par RPE à température ambiante en utilisant une méthode de compétition avec le piégeur de superoxyde BMPO (5-tert-butoxycarbonyl-5-methyl-1-pyrroline N-oxide) (Figure 1a). La source d'anion superoxyde est le sel de potassium (KO₂) utilisé à 5 mM dans le DMSO après dissolution assistée par les ultrasons. Les concentrations utilisées pour le composé (1) sont comprises entre 0,31 et 40 µM. Le piégeur BMPO est utilisé à la concentration finale de 25 mM. Ces expériences ont été réalisées avec un spectromètre RPE Miniscope MS 100 (Magnettech GmbH, Allemagne), fonctionnant à la fréquence de 9,5 GHz (bande X) avec les paramètres suivants : champ magnétique : 336,0 ± 9,9 mT ; temps de balayage : 20 s ; échantillonnage : 4096 points ; amplitude de modulation : 0,10 mT ; fréquence de modulation : 100 k κHz ; gain : 900 ; puissance micro-onde : 100 mW. L'inhibition de la formation d'adduit superoxyde du BMPO, traduite par une diminution d'intensité du spectre RPE de cette espèce, est dépendante de la dose de composé (1) appliquée et permet d'obtenir une concentration inhibitrice médiane (CI50) de 2,5 µM.

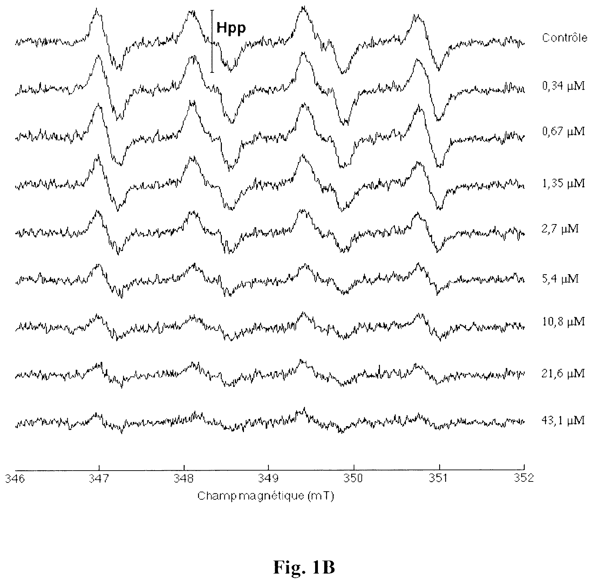

La réactivité du composé (1) avec l'anion superoxyde en phase aqueuse a également été étudiée par RPE à température ambiante en utilisant la même méthode de compétition avec le piégeur BMPO à 50 mM (Figure 1b). Dans ce cas, l'anion superoxyde est produit par la réaction enzymatique de la xanthine oxydase (XO, 53 mU/mL) avec la xanthine (X, 250 µM) dans le tampon PBS (pH 7,4) contenant 0,1 mM d'EDTA (acide éthylènediaminetétraacétique). Les concentrations utilisées pour le composé (1) sont comprises entre 0,34 µM et 43,1 µM. Ces expériences ont été réalisées avec un spectromètre RPE Elexsys E500 (Bruker, Wissembourg, France), fonctionnant à la fréquence de 9,82 GHz (bande X) avec les paramètres suivants : champ magnétique : 349,0 ± 6,6 mT ; temps de balayage : 41,94 s ; échantillonnage : 1024 points ; constante de temps : 40,96 ms ; temps de conversion : 40,96 ms ; amplitude de modulation : 0,10 mT ; fréquence de modulation : 100 kHz ; gain : 60 dB ; puissance micro-onde : 10 mW. L'inhibition de la formation d'adduit superoxyde du BMPO, traduite par une diminution d'intensité du spectre RPE de cette espèce après 10 min d'incubation, dépend de la concentration du composé (1) utilisée; on mesure une CI50 de 3,2 µ _{µµ µµ**µ} M.

Une réaction directe entre le composé (1) et l'adduit superoxyde du BMPO a été exclue par la vérification que la stabilité de l'adduit du BMPO, formé par préincubation avec le système X/XO, n'est pas modifiée en présence du composé (1) (résultat non présenté).

### EXEMPLE 3 : Réactivité du composé (1) avec l'anion superoxyde in vitro : étude par résonance paramagnétique électronique (RPE) de la disparition du signal de O₂°⁻ en présence du composé (1), et mise en évidence de la formation d'un intermédiaire radicalaire

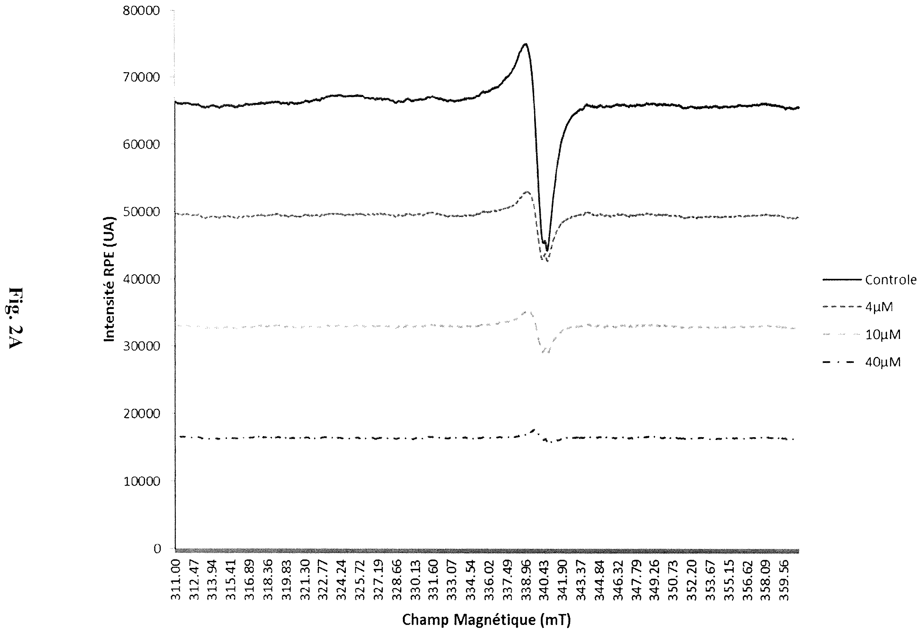

A fin de suivre l'interaction directe du composé (1) avec l'anion superoxyde, du superoxyde de potassium (KO₂) solide a été dissout par sonication dans le DMSO (concentration finale 2,5 mM), puis mélangé avec le composé (1) et immédiatement refroidi par immersion dans l'azote liquide. L'analyse RPE à 77 K (azote liquide) a été réalisée avec un spectromètre RPE Miniscope MS 100, fonctionnant à la fréquence de 9,5 GHz (bande X) avec les paramètres suivants : champ magnétique : 336,0 ± 49,8 mT ; temps de balayage : 20 s ; échantillonnage : 4096 points ; amplitude de modulation : 0,10 mT ; fréquence de modulation : 100 kHz ; gain : 900 ; puissance micro-onde : 6mW. Les résultats montrent une diminution du signal RPE de l'anion superoxyde, dépendante de la concentration en composé (1) (Figure 2a).

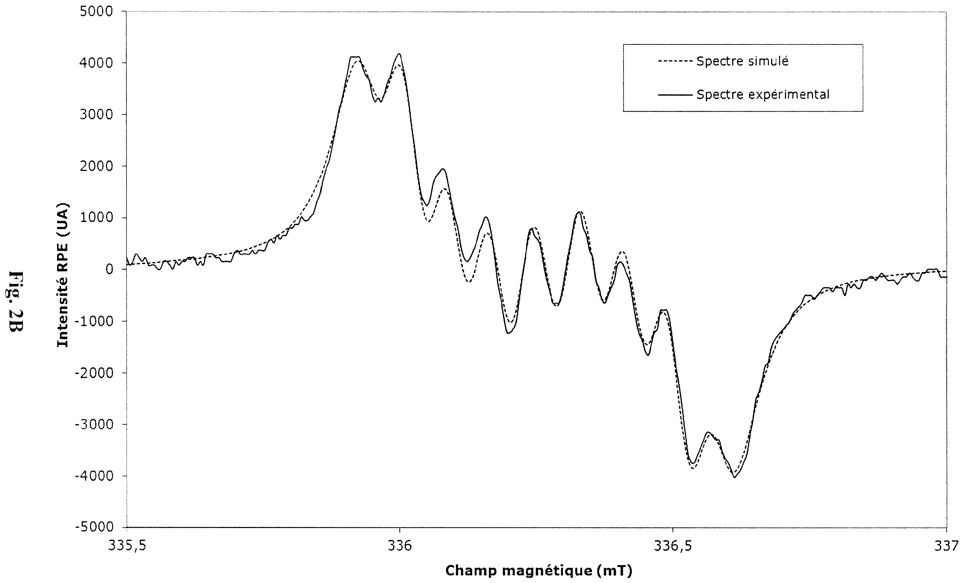

Le spectre RPE du radical formé par réaction du composé (1) (40 µM) avec l'anion superoxyde (0,5 mM KO₂ dissous dans le DMSO par sonication) a été enregistré à température ambiante (Figure 2b). Cette analyse RPE a été réalisée avec le spectromètre RPE Miniscope MS 100, fonctionnant à la fréquence de 9,5 GHz (bande X), avec les paramètres suivants : champ magnétique : 336,0 ± 1.9 mT ; temps de balayage : 20 s ; échantillonnage : 4096 points ; amplitude de modulation : 0,05 mT ; fréquence de modulation : 100 kHz ; gain : 900 ; puissance micro-onde :100 mW. Ce spectre de radical phénoxyle, centré autour de g=2,00 a été simulé grâce au logiciel de Rockenbauer et Korecz (Rockenbauer, A.; Korecz, L. Automatic computer simulations of ESR spectra. Appl. Magn. Reson. 10:29-43; 1996), en prenant en compte quatre constantes de couplage avec des protons, égales à : 0,33 ; 0,16 ; 0,08 et 0,08 mT. L'attribution la plus vraisemblable de ces couplages correspond à trois protons du système aromatique catéchol et à un proton du pyrrole. Les couplages à l'autre proton et à l'azote du noyau pyrrole ne sont pas résolus.

### EXEMPLE 4 : Formation d'une quinone intermédiaire par réaction du composé (1), avec l'anion superoxyde (O₂°⁻).

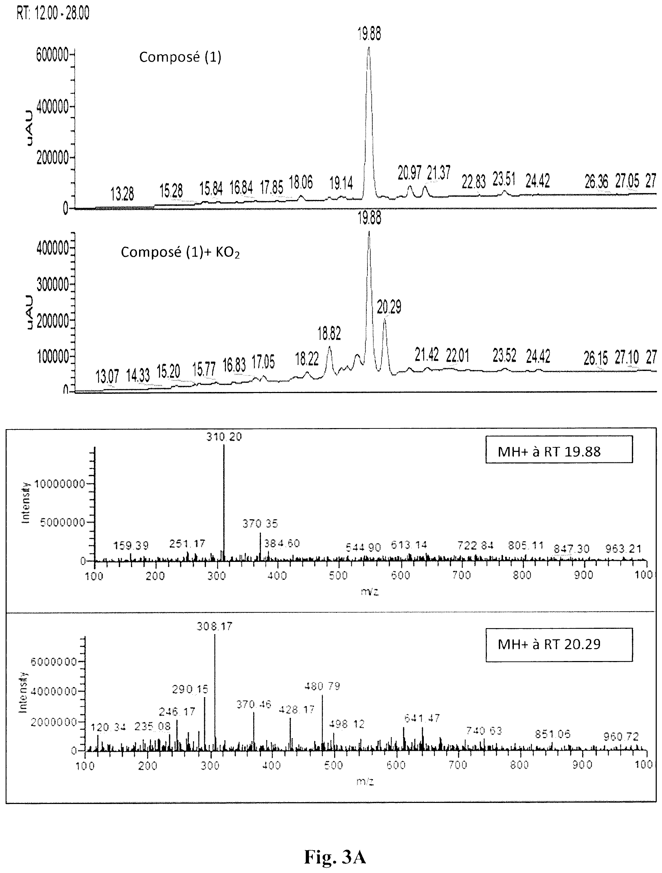

Le produit de l'oxydation du composé (*1*) par l'anion superoxyde en phase organique (KO₂ dissous par sonication dans le DMSO) a été caractérisé par HPLC (LC-Trap ; Surveyor, Thermo). Le mélange réactionnel (volume final 200µl) est constitué du composé (1) à 1,6 10⁻³M (20µl en DMSO), traité ou non avec du KO₂, 10µl (5mM). Le mélange réactionnel (20µl) a été injecté sur une colonne Gemini (C18, 2x150mm, 5µm, Phenomenex), avec initialement 98% solvant A (A=H₂O ; 0,1% acide formique), et 2% solvant B (acetonitrile ; 0,1% acide formique), à un débit de 0,2 ml/min. Après 5 min. le gradient est passé à 0% A et 100% B pendant 20 min. Les résultats obtenus (Figure 3a) montrent que le composé (1) de temps de rétention égal à 19.88, et de m/z =310,20 (MH+) est transformé partiellement en composés présentant entre autre un pic de rétention égal à 20,29 et de m/z = 308,17 (MH+). Ces résultats suggèrent l'oxydation du composé (1) par l'anion superoxyde.

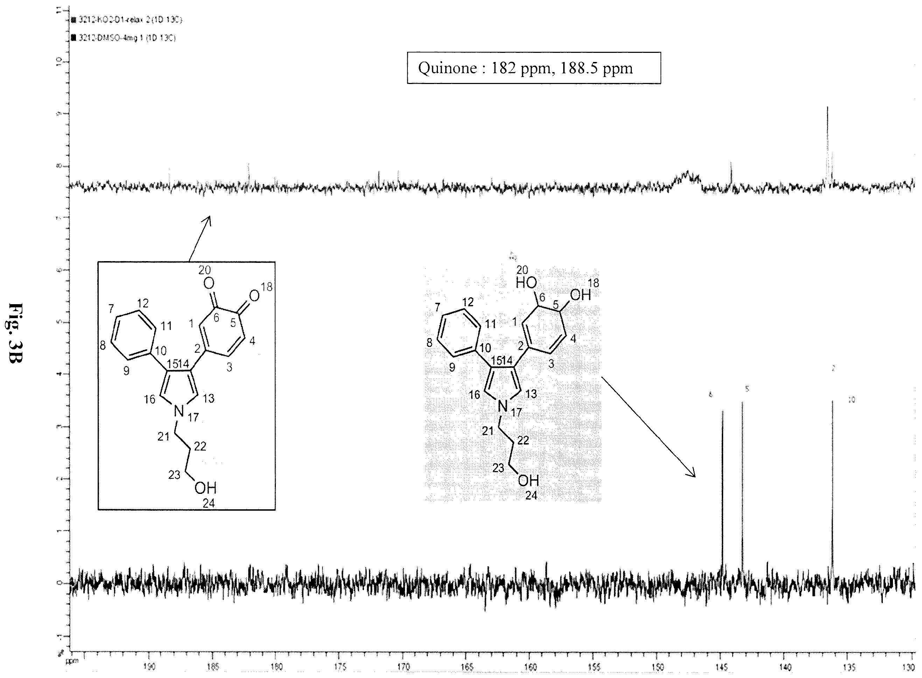

Le produit d'oxydation du composé (1) par l'anion superoxyde généré par le KO₂ a été analysé par RMN ¹³C, sur un spectromètre Brucker AVANCE-500. Les résultats (Figure 3b) nous montrent la suppression des signaux correspondant aux carbones 5 et 6 du composé (1) et la formation de deux signaux à un déplacement chimique de 182 ppm et 188.5 ppm, caractéristiques des quinones pour les deux carbones 5 et 6 du composé (1) traité.

### EXEMPLE 5 : Formation d'un composé dimérique MH⁺=631 « in vitro » par réaction du composé (1), avec l'anion superoxyde (O₂°⁻) ou à pH=pKa=8.89

La formation du composé dimérique a été obtenue après réaction du composé (1) (0,3 10⁻³M) avec de l'anion superoxyde généré par le complexe xanthine-xanthine oxydase, dans un solvant PBS-EDTA.

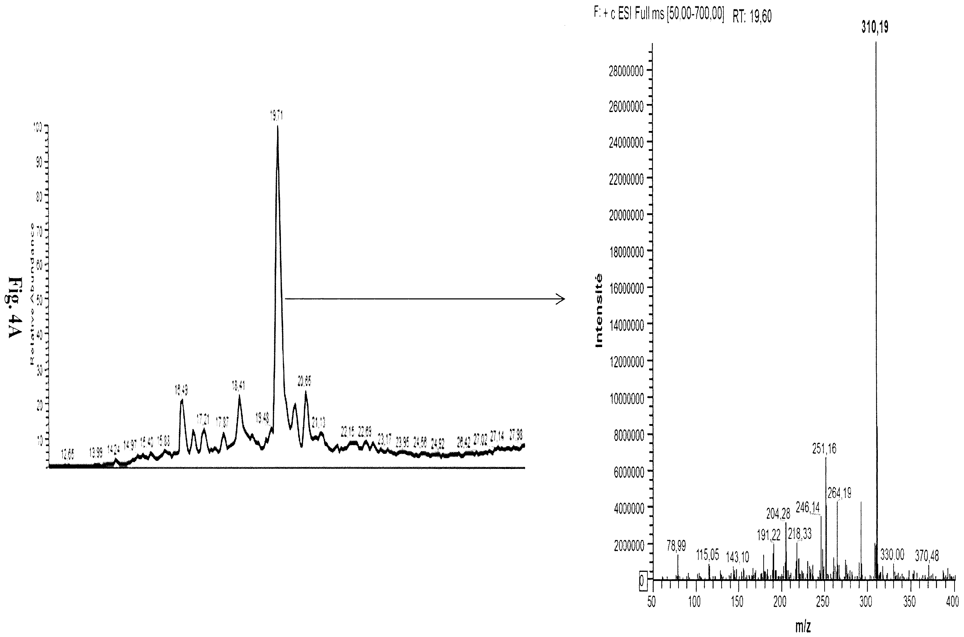

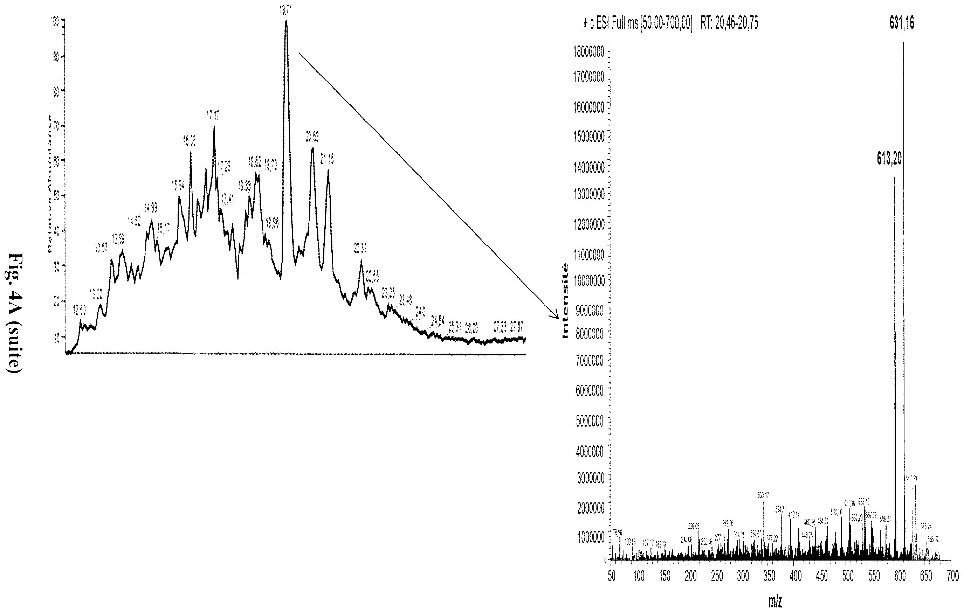

Le suivi de la formation du composé dimérique MH+ 631 a été déterminé sur une HPLC Surveyor (Thermo), couplée avec un LCQ deca (Thermo). Le mélange réactionnel (10µl) a été injecté sur une colonne Gemini (C18, 2x150mm, 5µm, Phenomenex), avec initialement 98% solvant A (A=H₂O ; 0,1% acide formique), et 2% solvant B (acetonitrile ; 0,1% acide formique), à un débit de 0,2ml/min. Après 5 min. le gradient est passé à 0%A, 100%B en 20 min. Les résultats obtenus (Figure 4a) montrent que le composé (1) de temps de rétention égal à 19.71, et de m/z =310,19 (MH+) est transformé en composés présentant entre autre un pic de rétention égal à 20,63 et de m/z = 631,16 (MH+). En présence de superoxyde dismutase et de catalase, les composés dimériques ne sont pas formés (résultats non montrés). Ces résultats sont en faveur de la dimérisation du composé (1) en présence d'anion superoxyde.

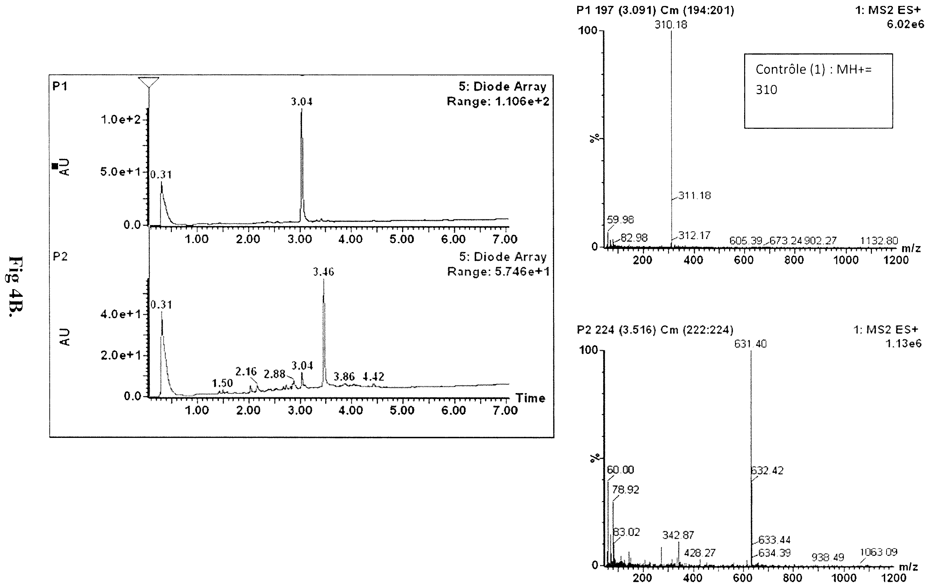

Afin de réaliser la dimérisation du composé (1) par couplage oxydatif au pH égal à la valeur du pKa du composé, le pKa théorique a été déterminé par des calculs quantiques en utilisant le logiciel Gaussian03 (Gaussian, Inc., Wallingford CT, 2004) et une méthode adaptée à partir de Liptak et al. (J. Am. Chem. Soc. 2002, 124, 6421-6427), notamment en modifiant les valeurs par défaut des paramètres PCMDOC, RADII, SCFVAC et TSARE pour les rendre compatibles avec les valeurs de Gaussian98, logiciel utilisé dans la publication d'origine. La valeur du pKa obtenue par cette méthode a été de 8.89, valeur qui a été utilisée ensuite pour ajuster le pH d'une solution de Tris-HCl 0,15M, DMSO :10%, pour la réaction de dimérisation du composé (1) à 0,3 10⁻² M final. Le mélange réactionnel (10µl) a été chromatographié par UHPLC (Waters, TQD, PDA, ELSD), sur une colonne HSS C18 (Waters, 2,1x50mm ; 1,8µm) par un gradient de solvant contenant initialement 95% (A : H2O, 0,1% acide formique) et 5% (B: acétonitrile, 0,1% acide formique) puis 100% de solvant (B) en 6min30sec, à un débit de 0,6ml par min. Les résultats présentés dans la Figure 4b, montrent que le composé (1), présentant un pic unique à un temps de rétention (RT) de 3,04 et de m/z = 310,19 (correspondant à l'ion MH+) est transformé en un composé dont le temps de rétention est de RT=3,46 et de m/z = 631,40 (MH+). Ces résultats suggèrent que le composé (1) s'est oxydé et dimérisé.

Afin d'obtenir le dimère MH+ 631 en quantité suffisante pour déterminer sa structure, le couplage oxydatif de 12 mg du composé (1) dans une solution de Tris-HCl, 0.15M, pH 9.4, DMSO (15% final) a été réalisé. Après 48h, la réaction a été stoppée par quelques gouttes d'HCl 0.1N. Le dimère MH+ 631 a été séparé sur colonne C18, HPLC préparative (ACN, H₂O, 0.1% acide formique), suivi d'une extraction en présence d'un mélange Acetonitrile (50%) éther (50%). Après évaporation, le composé dimérique a été dissous dans de l'acetonitrile deutéré, et analysé par RMN (1H, 13C, cosy, HMBC, HSQC) (Brucker 600) :
RMN ¹H (CD₃CN; 263 K; 600 MHz) *δ*(ppm) ∼ 7,5 (1 H; sl); 7,33 (2 H; m); 7,26 (3 H; m); 7,17 (2 H; m); 7,13 (2 H; m); 7,05 (1 H; m); ∼ 7,0 (2 H; sl); 6,78 (2 H; m); 6,77 (1 H; s); 6,55 (1 H; s); 6,51 (1 H; d; *J* = 2 Hz); 6,42 (1 H; d; *J* = 2 Hz); 6,02 (1 H; s); 3,87 (2 H; t; *J* = 7,2 Hz); 3,84 (2 H; t; *J* = 7,2 Hz); 3,42; (2 H; t; *J* = 5,4 Hz); 3,31; (2 H; t; *J* = 5,4 Hz); 3,05 (1 H; sl); 2,97 (1 H; sl); 1,82; (2 H; quint; *J* = 6,6 Hz); 1,70; (2 H; quint; *J* = 6,6 Hz).
RMN ¹³C (CD₃CN; 263 K; 150 MHz) *δ* (ppm) 188,1; 182,8; 151,6; 145,1; 144,3; 143,1; 137,1; 136,1; 129,8; 129,54; 129,48; 129,4; 129,1; 128,8; 128,2; 127,3; 126,0; 125,4; 124,0; 123,8; 123,3; 123,1; 123,0; 122,6; 122,2; 120,6; 118,1; 114,5; 58,9; 58,8; 47,1; 46,7; 34,9; 34,2.

La structure proposée pour le dimère est la suivante :

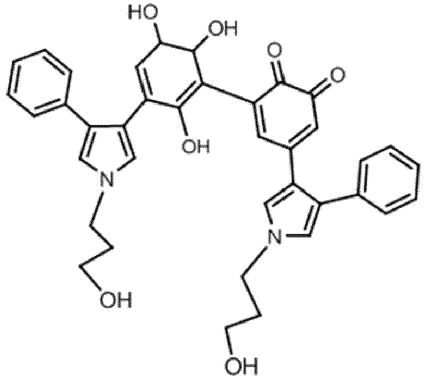

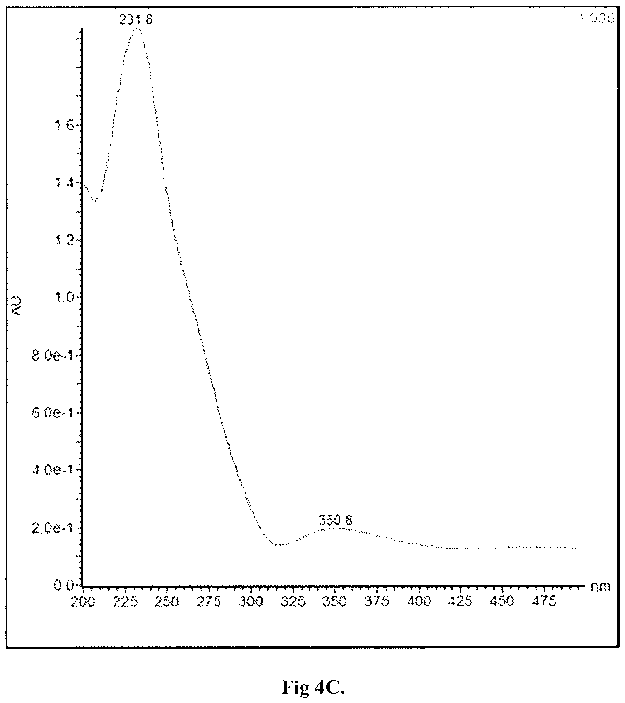

La détermination de la masse exacte du dimère (MH+ :631.2428) (C38 H35 N2 O7) a été réalisée sur un spectrometre de masse à temps de vol de type LCT premier (Waters). Le spectre UV du composé dimérique a été extrait du chromatogramme obtenu sur une chaine Acquity (Waters) équipée d'un détecteur UV à barrette de diodes (Acquity PDA detector)(figure 4C)

### EXEMPLE 6 : Etude in ovo de l'effet du composé (1) sur l'angiogenèse tumorale

L'effet du composé *(1)* sur l'angiogenèse tumorale a été évalué à l'aide du modèle *in ovo* basé sur la greffe de tumeur humaine sur la membrane chorioallantoïdienne de l'embryon de poulet (CAM). Cette approche expérimentale permet en quelques jours seulement d'évaluer la progression tumorale et le niveau de vascularisation de la tumeur.

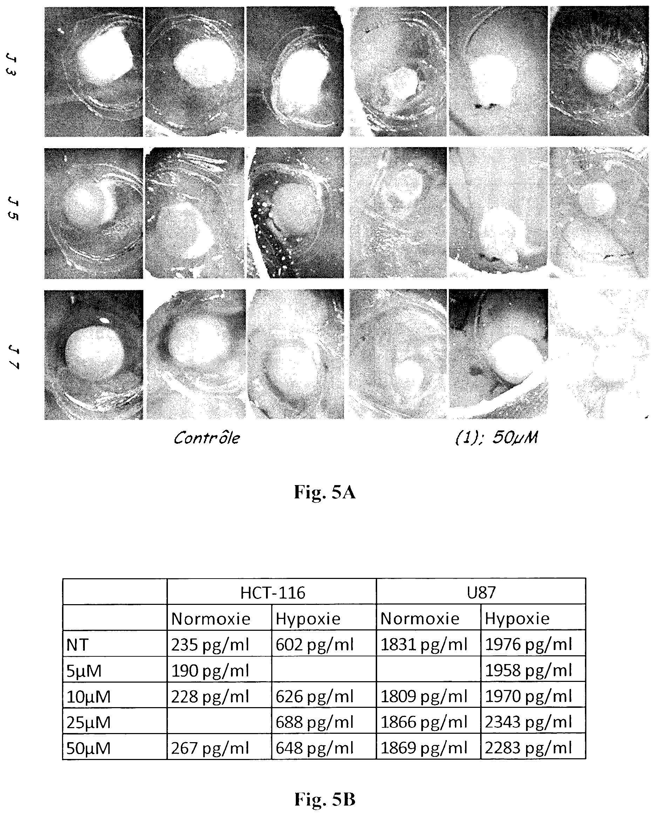

Au dixième jour du développement embryonnaire des cellules de glioblastome (U87, 5x10⁶ dans 20 µl) ont été déposées sur la membrane chorioallantoïdienne. Deux jours après la greffe, le composé *(1)* à la concentration 50 µM dans un volume de 20 µl a été déposé sur la surface de la tumeur formée. Ce traitement a été répété toutes les 24 heures pendant 5 jours. Le groupe contrôle a été traité avec le solvant. Le test a été fait sur 20 œufs porteurs de tumeurs de glioblastome pour chaque groupe. Les images des tumeurs ont été prises au jour 3 (24h après traitement), au jour 5 (3 jours après traitement) et au jour 7 (5 jours après traitement) de développement tumoral. Les résultats présentés dans la Figure 5a, montrent une inhibition de la vascularisation tumorale déjà après 3 traitements successifs (jour 5), comparé au groupe contrôle. Cette réduction de la vascularisation tumorale observée également au jour 7 du développement tumoral est associée à une inhibition significative de la croissance de la tumeur traitée. Ces résultats mettent en évidence une activité anti-angiogénique péri-tumorale et antitumorale du composé *(1)*. Afin de déterminer le mécanisme anti-angiogenique du composé (*1*), le dosage du VEGF a été évalué par la technique ELISA (invitrogen, Novex technology), à partir de surnageant de culture de cellules U87 et HCT-116, en condition de normoxie et d'hypoxie. Les résultats indiquent (Figure 5b) que le composé (*1*) aux concentrations de 5 à 50 µM, ne modifie pas la secretion du VEGF après 24h de traitement des cellules. Ces résultats suggèrent que le mécanisme d'inhibition de l'angiogenèse peri tumorale du composé *(1)* pourrait être une action directe sur les cellules endothéliales.

### EXEMPLE 7 : Etude in ovo de l'effet du composé (1) sur l'angiogenèse physiologique, sur un réseau vasculaire déjà formé.

L'effet du composé *(1)* sur l'angiogenèse physiologique a été évalué à l'aide du modèle de la membrane chorioallantoïdienne de l'embryon de poulet (CAM) qui constitue un tissu richement vascularisé.

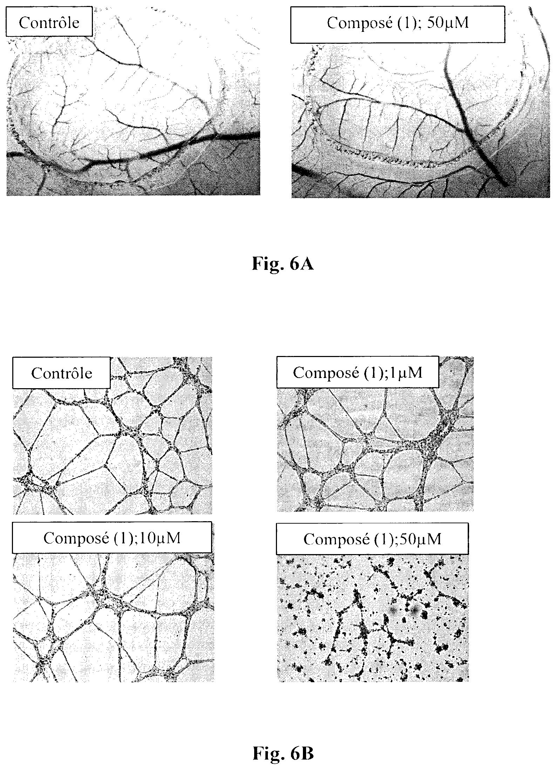

Au septième jour du développement embryonnaire 20 µl du solution du composé *(1)* à 50 µM ont été déposés sur la membrane chorioallantoïdienne alors que les membranes contrôles ont été traitées avec le solvant. Les résultats présentés dans la Figure 6a, montrent l'absence d'effet du composé *(1)* sur l'angiogenèse physiologique, comparé au groupe contrôle. Ces résultats suggérent que le composé *(1)* ne semble pas agir comme une drogue anti vasculaire, ne modifiant pas le réseau vasculaire déjà formé.

### EXEMPLE 8: Etude in vitro de l'effet du composé (1) sur la formation de tube vasculaire « de novo »

Afin de suivre l'effet du composé (*1*) sur la formation « de novo » de tubes vasculaires, des cellules endothéliales humaines (HUVEC), ont été cultivées dans une matrice (matrigel), en présence ou non du composé *(1)*. Après 24h, l'effet du composé *(1)* est analysé par microscopie optique et illustré par des photographies. Les résultats (Figure 6b) montrent que le composé (*1*) prévient la formation de capillaires (tubes formés à partir des cellules endothéliales). Le composé *(1)* agit donc directement sur les cellules endothéliales en croissance, en bloquant la formation des capillaires, contrairement à l'absence d'effet sur un réseau capillaire déjà formé, à l'état « quiescent ».

### EXEMPLE 9 : Etude in vitro de l'effet du composé (1) sur les cellules endothéliales quiescentes ou en croissance.

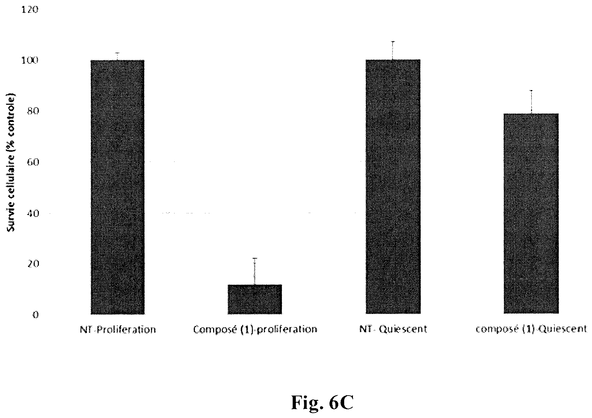

Afin de comprendre la différence d'effets observés du composé *(1)* selon que le réseau vasculaire soit déjà formé ou en croissance « de novo » , des cellules endothéliales vasculaires ont été mises en culture à deux densités cellulaires différentes de façon à obtenir des cellules en phase de prolifération ou des cellules quiescentes, puis traitées ou non par le composé *(1)* , à 10µM (valeur de l'IC50 : 8µM) pendant 48h. Les résultats de la figure 6c montrent que le composé *(1)* agit uniquement sur les cellules endothéliales en phase de prolifération (78% inhibition). Ce résultat permet donc de définir une specificité d'action du composé *(1)* vis-à-vis des cellules endothéliales en phase de prolifération intensive comme au cours de l'angiogenèse péri tumorale, la formation « de novo » de tubes vasculaires ou la prolifération de cellules endothéliales en culture. Le composé *(1)* pourrait donc être utilisé pour le traitement des pathologies associées à une angiogenèse excessive et anormale, comme le cancer ou la DMLA.

### EXEMPLE 10 : Etude biologique in vitro, sur cellules cancéreuses

L'activité biologique du composé (*1*), ainsi que celle de ses analogues structuraux a été étudiée *in vitro* sur 7 lignées cellulaires cancéreuses différentes:
- HCT-116 (carcinome colorectal)
- U87 (glioblastome)
- K562 (leucémie myéloïde) et K562 R
- MDA-MB 231 (adénocarcinome mammaire)
- MCF-7 (adénocarcinome mammaire)
- A549 (adénocarcinome alvéolaire pulmonaire)

Les cellules sélectionnées pour cette étude ont été incubées à 37°C en présence du composé *(1)* ajouté dans le milieu de culture à différentes concentrations et à différents temps. L'ensemble des expériences réalisées a permis de déterminer le degré de la cytotoxicité du composé testé (IC50), sa capacité à induire une mort cellulaire par apoptose, en étroite dépendance vis-à-vis de l'anion superoxyde extracellulaire pour son activation.

### 1. Etude de l'effet du composé (1) sur la croissance cellulaire

L'effet du composé *(1)* sur la croissance des cellules HCT-116, U87, K562 , K562R, MDA-MB 231, MCF-7 et A549, cultivées en normoxie, a été évaluée à l'aide d'un test de croissance cellulaire (CellTiter-Blue™ Cell Viability Assay, Promega).

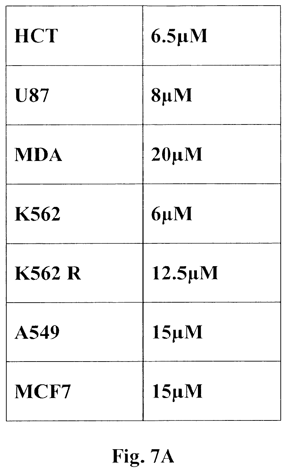

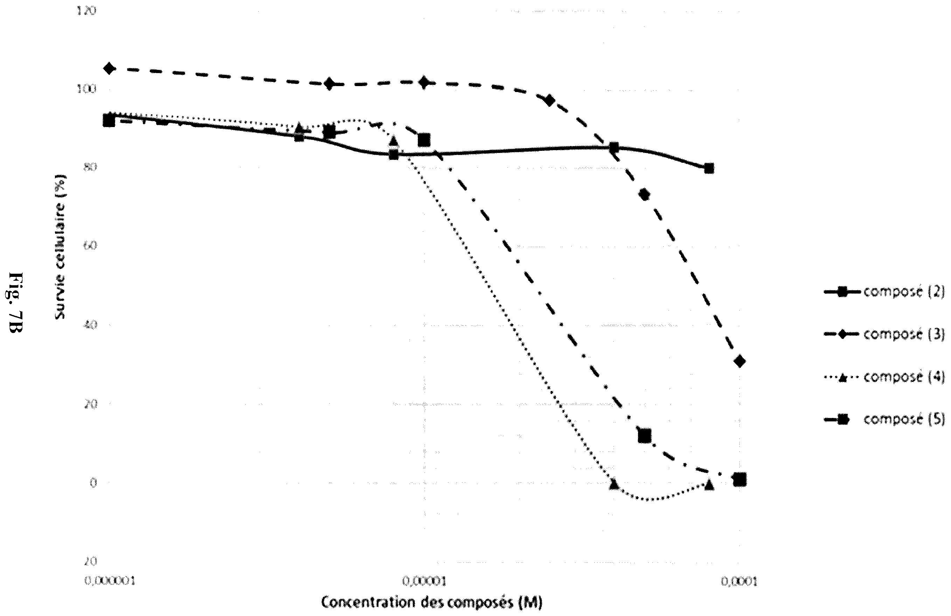

Les résultats présentés dans la Figure 7a montrent les valeurs de l'IC50 (concentration du composé qui induit la diminution de la croissance cellulaire de 50%) déterminé après 72h du traitement avec le composé *(1)*. Elle est de 7,5µM pour les cellules HCT-116, 7µM pour les U87, 20µM pour les MDA-MB 231, 6µM pour les K562, 12,5µM pour les K562R, 15µM pour les MCF-7 et 15µM pour les A549. Les résultats présentés dans la Figure 7b montrent l'effet cytotoxique dose dépendant des composés (*2, 3, 4*, *et 5*) sur les cellules HCT-116 cultivées en normoxie pendant 72h. L'absence de catéchol entraine la perte de la cytotoxicité des composés 2 et 3, alors que la modification de la chaine latérale par une chaine butyl (5) ou o-ethoxy (4) ne modifie pas sensiblement la valeur de l'IC50.

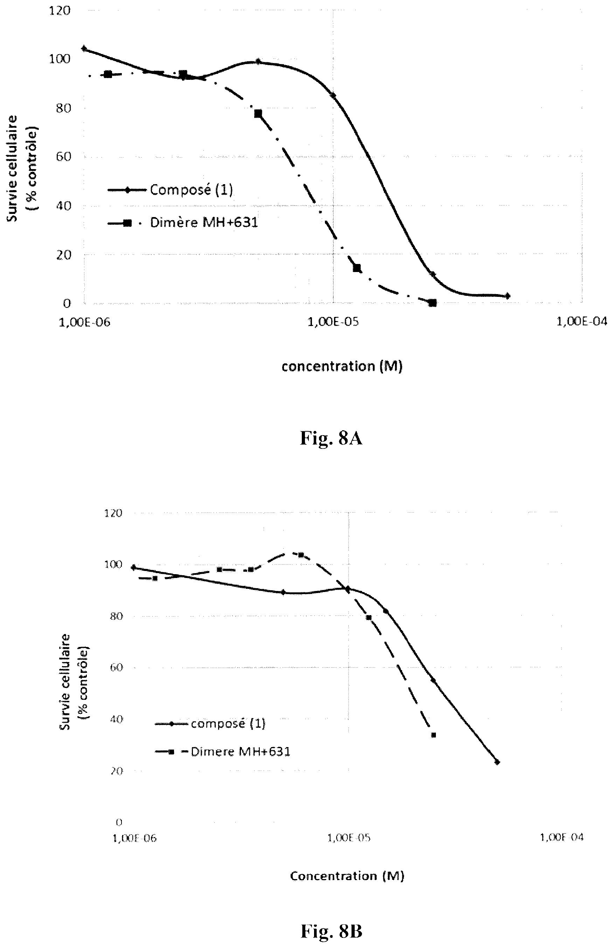

Les résultats présentés dans la Figure 8a montrent l'effet cytotoxique comparatif du composé *(1)* et du dimère MH+631 préalablement obtenu par couplage oxydatif du composé *(1)* sur les cellules HCT-116 cultivées en normoxie, pendant 48h. Le dimère est toxique, avec une IC50 de 7,5µM, moitié de celle du composé *(1)*, en raison du poids moléculaire double du dimère (PM=630d). Les résultats présentés dans la Figure 8b montrent l'effet cytotoxique comparatif du composé *(1)* et du dimère MH+631 sur les cellules HCT-116 cultivées en hypoxie (2% O₂) pendant 48h. Le dimère est toxique, avec une IC50 de 10µM, environ la moitié de celle du composé *(1)*, en raison du poids moléculaire double du dimère.

### 2. Etude de l'effet du composé (1) sur l'induction de l'apoptose en normoxie

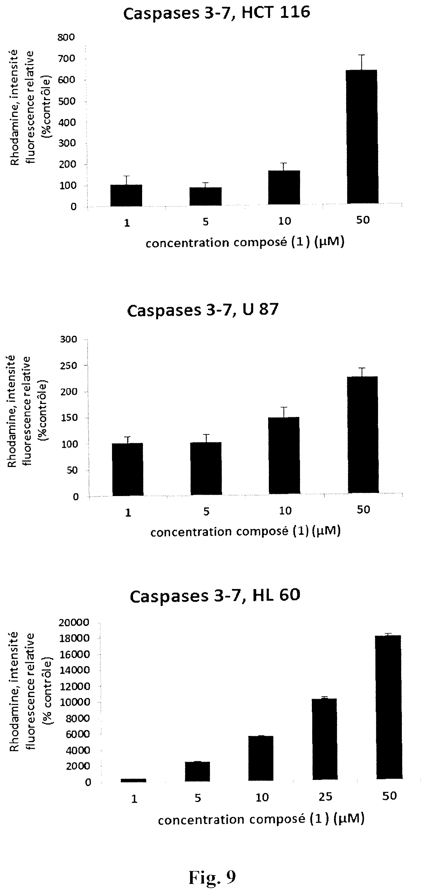

Afin de préciser si le composé *(1)* entraîne une mort cellulaire par apoptose, l'activité des caspases 3 et 7, enzymes marqueurs de l'apoptose, a été mesurée à l'aide du test Apo-ONE (Promega) dans les cellules HCT-116, U87 et HL60 traitées en normoxie pendant 24h avec le composé *(1)* aux concentrations variant de 1µM à 50µM. Les résultats présentés dans la Figure 9 montrent que le traitement de cellules avec le composé *(1)* induit une activation significative de deux caspases, quel que soit la lignée cellulaire. La plus forte activation a été obtenue avec les cellules HL60.

### 3. Suppression du signal de l'anion superoxyde cellulaire par le composé (1)

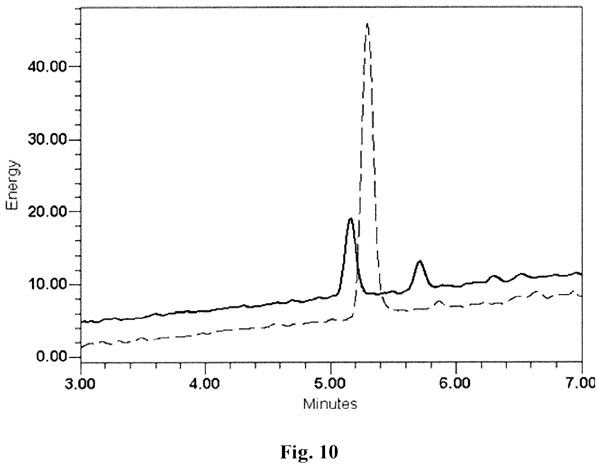

L'effet du composé *(1)* sur l'anion superoxyde a été étudié suite au traitement des cellules HCT-116 avec 50µM du composé *(1)* durant 3h et suivi par l' incubation avec la sonde dihydroéthidium (DHE) (10µM) pendant 20 minutes. Après retrait du milieu de culture et lavage au DPBS glacé, les cellules ont été récupérées dans 1ml de DPBS froid. Après centrifugation à 1000g pendant 5 minutes, le culot a été repris dans 150µl de PBS avec Triton X100 (0.1%), agité et centrifugé à 1000g pendant 5 minutes. Le surnageant a été mélangé avec une solution de 0.2M HClO₄ dans du méthanol, agité et placé dans la glace pendant 2 heures. Après centrifugation à 20000g pendant 30 minutes, le surnageant est analysé par HPLC couplé à la fluorescence (λex = 510nm, λem = 595nm). Dans les cellules non traitées (trait continu), le premier pic à t_{R}= 5,16 min correspond à la forme oxydée « E+ », produit d'oxydation par les peroxydases et le second pic à t_{R}= 5,71 min à la forme oxydée 2-OH E+, spécifique de l'interaction avec l'anion superoxyde. Dans les cellules traitées, le premier pic à t_{R}= 5,29 min correspondant à la forme oxydée « E+ » est augmenté, alors que le second pic à t_{R}= 5,71 min, correspondant à la forme oxydée 2-OH E+, spécifique de l'interaction avec l'anion superoxyde a été supprimé. Les résultats obtenus montrent une inhibition du signal 2OH E+ correspondant à la forme oxydée de la DHE par l'anion superoxyde, et une augmentation du signal E+ correspondant à l'oxydation de la DHE par les peroxydases ou catalase, activée par H₂O₂. (Figure 10).

### 4. Dépendance du composé (1) de l'anion superoxyde extracellulaire/membranaire pour l'action cytotoxique et pro-apoptotique

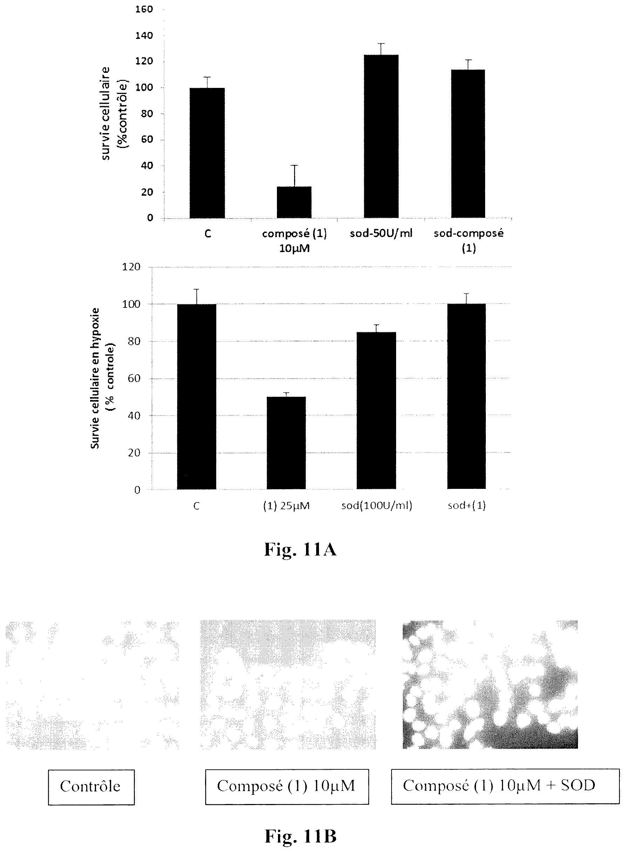

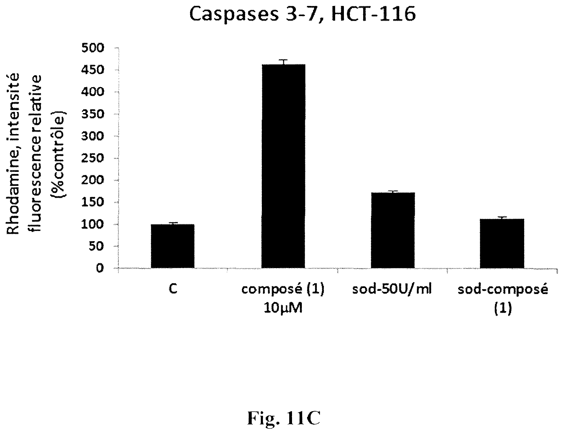

Afin de déterminer le rôle de l'anion superoxyde, produit par les cellules, dans l'activation du composé *(1)*, les cellules HCT-116 ont été prétraitées pendant 1h avec l'enzyme superoxyde dismutase (SOD1) à 50 U/ml, puis traitées avec le composé *(1)*. Les cellules ont été analysées pour évaluer d'une part la survie cellulaire en normoxie (72h traitement) et en hypoxie (48h traitement). D'autre part, la formation de chromatine condensée en normoxie et l'activité des caspases 3 et 7 en normoxie ont été évaluées. Les résultats présentés dans la Figure 11A montrent que la supression d'anion superoxyde suite au traitement par la SOD, bloque totalement l'effet cytotoxique du composé *(1)* à 10µM en normoxie et à 25µM en hypoxie. De même, le prétraitement par la SOD supprime totalement l'augmentation de la chromatine condensée en normoxie visualisée après coloration par le Hoechst 33258 (Figure 11 B). De la même façon, l'action du composé (1) sur l'activation des caspases 3-7 en normoxie est totalement supprimée par l'action de SOD (Figure 11 C). Ces résultats suggèrent que le composé *(1)* est une prodrogue activable spécifiquement par l'anion superoxyde.

## Revendications

1. Composé de formule (I) suivante : 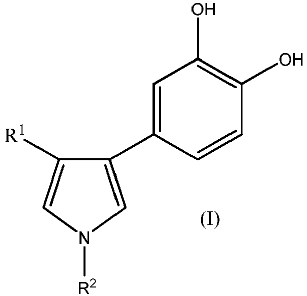 dans laquelle :
• R¹ représente un groupe aryle, éventuellement substitué par un ou plusieurs groupes (C₁-C₂)alkyle, un ou plusieurs halogènes, un ou plusieurs groupes -OH, -CN ou CF₃, et une combinaison de ceux-ci ; et
• R² représente un groupe (C₁-C₆)alkyle ou un groupe hydroxy(C₁-C₆)alkyle ou un groupe (C₁-C₄)alcoxy(C₁-C₆)alkyle ;
ou un hydrate ou solvate pharmaceutiquement acceptable de celui-ci.

2. Composé selon la revendication 1, **caractérisé en ce que** R¹ représente un phényle et R² représente un groupe (C₁-C₆)alkyle ou un groupe hydroxy(C₁-C₆)alkyle ou un groupe (C₁-C₄)alcoxy(C₁-C₆)alkyle.

3. Composé selon la revendication 1 ou 2, choisi parmi les composés de formule (Ia), (Ib) et (Ic) suivantes : 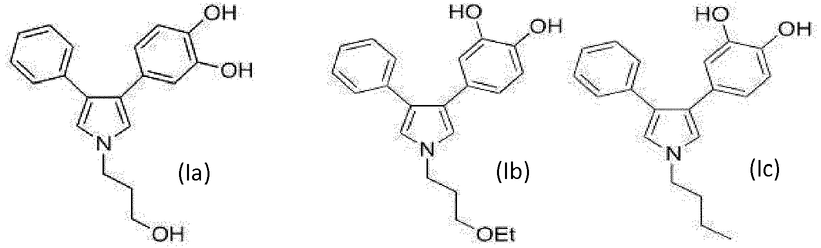 et leurs hydrates et solvates pharmaceutiquement acceptables.

4. Composé selon l'une quelconque des revendications précédentes, pour son utilisation en tant que médicament.

5. Composé pour son utilisation selon la revendication 4, pour son utilisation en tant que prodrogue.

6. Composé de formule générale (IV) : 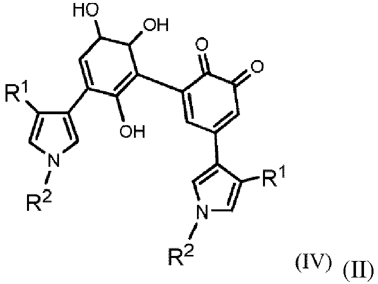 dans laquelle R¹ et R² sont tels que définis dans l'une quelconque des revendications 1 ou 2.

7. Composé de formule générale (IV) telle que définie en revendication 6 pour son utilisation en tant que médicament.

8. Composé de formule (I) ou (IV) selon l'une quelconque des revendications précédentes, pour son utilisation dans le traitement du cancer et des pathologies associées à une angiogenèse excessive.

9. Composition pharmaceutique comprenant à titre de prodrogue au moins un composé de formule (I) ou (IV) selon l'une quelconque des revendications 1 à 8 et un excipient pharmaceutiquement acceptable.

10. Composition pharmaceutique selon la revendication 9, pour son utilisation dans le traitement du cancer et des pathologies associées à une angiogenèse excessive.

11. Procédé de préparation d'un composé selon l'une quelconque des revendications 1 à 3, comprenant la réaction de condensation d'un composé de formule (V) suivante : 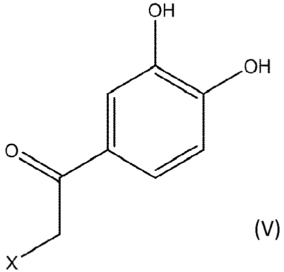 dans laquelle X est un halogène, en particulier un atome de chlore,
avec un composé de formule (VI) et un composé de formule (VII) suivantes :
R²-NH₂ (VI)
R¹-CH₂-CHO (VII)
dans laquelle R¹ et R² sont tels que défini dans la revendication 1.

## Patentansprüche

1. Verbindung der folgenden Formel (I), 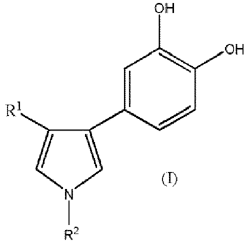 wobei:
• R¹ eine Arylgruppe darstellt, gegebenenfalls substituiert durch eine oder mehrere (C₁-C₂)-Alkylgruppen, ein oder mehrere Halogene, eine oder mehrere -OH-, -CN- oder CF₃-Gruppen und eine Kombination davon; und
• R² eine (C₁-C₆)-Alkylgruppe oder eine Hydroxy-(C₁-C₆)-Alkylgruppe oder eine (C₁-C₄)-Alkoxy-(C₁-C₆)-Alkylgruppe darstellt; oder
ein pharmazeutisch verträgliches Hydrat oder Solvat davon.

2. Verbindung nach Anspruch 1, **dadurch gekennzeichnet, dass** R¹ eine Phenylgruppe darstellt und R² eine (C₁-C₆)-Alkylgruppe oder eine Hydroxy-(C₁-C₆)-Alkylgruppe oder eine (C₁-C₄)-Alkoxy-(C₁-C₆)-Alkylgruppe darstellt.

3. Verbindung nach Anspruch 1 oder 2, ausgewählt aus den folgenden Verbindungen der Formel (Ia), (Ib) und (Ic): 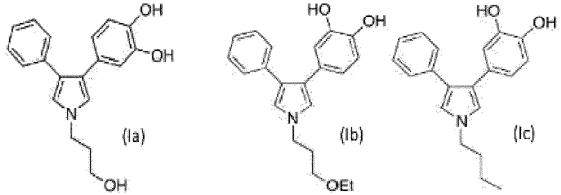 und ihren pharmazeutisch verträglichen Hydraten und Solvaten.

4. Verbindung nach einem der vorhergehenden Ansprüche zur Verwendung als Medikament.

5. Verbindung zur Verwendung nach Anspruch 4 zur Verwendung als Prodrug.

6. Verbindung der allgemeinen Formel (IV): 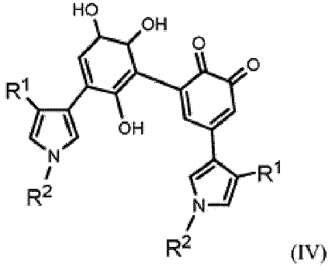 wobei R¹ und R² wie in einem der Ansprüche 1 oder 2 definiert sind.

7. Verbindung der allgemeinen Formel (IV) wie in Anspruch 6 definiert zur Verwendung als Medikament.

8. Verbindung der Formel (I) oder (IV) nach einem der vorhergehenden Ansprüche zur Verwendung bei der Behandlung von Krebs und Pathologien, die mit einer übermäßigen Angiogenese verbunden sind.

9. Pharmazeutische Zusammensetzung aufweisend als Prodrug mindestens eine Verbindung der Formel (I) oder (IV) nach einem der Ansprüche 1 bis 8 und einen pharmazeutisch verträglichen Träger.

10. Pharmazeutische Zusammensetzung nach Anspruch 9 zur Verwendung bei der Behandlung von Krebs und Pathologien, die mit einer übermäßigen Angiogenese verbunden sind.

11. Verfahren zur Herstellung einer Verbindung nach einem der Ansprüche 1 bis 3, aufweisend die Kondensationsreaktion einer Verbindung der folgenden Formel (V): 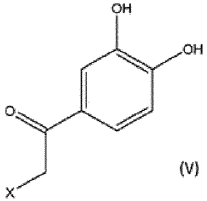 wobei X ein Halogen, insbesondere ein Chloratom ist,
mit einer folgenden Verbindung der Formel (VI) und einer folgenden Verbindung der Formel (VII):
R²-NH₂ (VI)
R¹-CH₂-CHO (VII)
wobei R¹ und R² wie in Anspruch 1 definiert sind.

## Claims

1. Compound of the following formula (I): 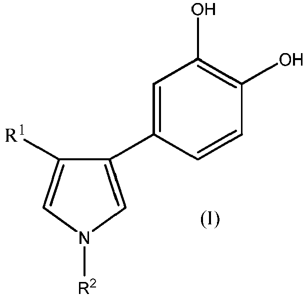 wherein:
• R¹ represents an aryl group, optionally substituted by one or more (C₁-C₂)alkyl groups, one or more halogens, one or more -OH, -CN or CF₃ groups, and a combination thereof; and
• R² represents a (C₁-C₆)alkyl group or a hydroxy(C₁-C₆)alkyl group or a (C₁-C₄)alkoxy(C₁-C₆)alkyl group;
or a pharmaceutically acceptable hydrate or solvate thereof.

2. Compound according to claim 1, **characterized in that** R¹ represents a phenyl and R² represents a (C₁-C₆)alkyl group or a hydroxy(C₁-C₆)alkyl group or a (C₁-C₄)alkoxy(C₁-C₆)alkyl group.

3. Compound according to claim 1 or 2, selected from the compounds of the following formulae (Ia), (Ib) and (Ic): 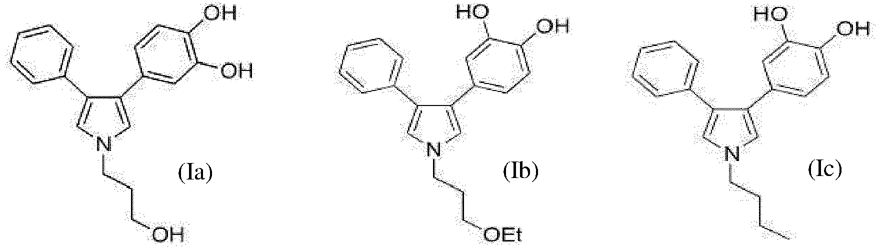 and the pharmaceutically acceptable hydrates and solvates thereof.

4. Compound according to any one of the preceding claims, for use as a drug.

5. Compound for use according to claim 4, for use as a prodrug.

6. Compound of formula (IV): 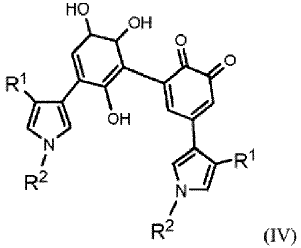 In which R¹ and R² are defined as in anyone of claims 1 or 2.

7. Compound of formula (IV) as defined in claim 6 for use as a drug.

8. Compound of formula (I) or (IV) according to any one of the preceding claims, for use to treat cancer and diseases associated with excessive angiogenesis.

9. Pharmaceutical composition comprising as prodrug at least one compound of formula (I) or (IV) according to any one of claims 1 to 8 and a pharmaceutically acceptable excipient.

10. Pharmaceutical composition according to claim 9, for use to treat cancer and diseases associated with excessive angiogenesis.

11. Method for preparing a compound according to any one of claims 1 to 3, comprising the condensation reaction of a compound of the following formula (V): 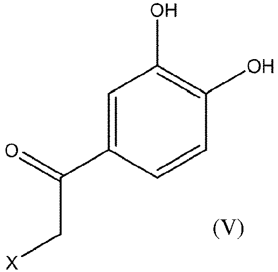 wherein X is a halogen, in particular a chlorine atom,
with a compound of the following formula (VI) and a compound of the following formula (VII):
R²-NH₂ (VI)
R¹-CH₂-CHO (VII)
wherein R¹ and R² are as defined in claim 1.
